(19) Europäisches Patentamt / European Patent Office / Office européen des brevets

(11) **EP 3 835 972 B1**

(12) **EUROPEAN PATENT SPECIFICATION**

(45) Date of publication and mention
of the grant of the patent:
**16.08.2023 Bulletin 2023/33**

(21) Application number: **19860100.7**

(22) Date of filing: **03.07.2019**

(51) International Patent Classification (IPC):
$G16H\ 50/20^{(2018.01)}$    $G16H\ 50/30^{(2018.01)}$
$G16H\ 50/70^{(2018.01)}$    $G16H\ 20/70^{(2018.01)}$
$G06F\ 16/332^{(2019.01)}$    $G06F\ 16/33^{(2019.01)}$
$A61B\ 5/00^{(2006.01)}$    $G06F\ 40/35^{(2020.01)}$
$G06F\ 40/216^{(2020.01)}$    $G06F\ 40/284^{(2020.01)}$
$G06F\ 40/268^{(2020.01)}$    $G06F\ 40/30^{(2020.01)}$

(52) Cooperative Patent Classification (CPC):
**A61B 5/7275; A61B 5/4088; A61B 5/4803;
A61B 5/7267; G06F 16/3329; G06F 16/3347;
G06F 40/216; G06F 40/268; G06F 40/284;
G06F 40/30; G06F 40/35; G16H 20/70;
G16H 50/20; G16H 50/30; G16H 50/70**

(86) International application number:
**PCT/JP2019/026484**

(87) International publication number:
**WO 2020/054186 (19.03.2020 Gazette 2020/12)**

(54) **COGNITIVE IMPAIRMENT PREDICTION DEVICE, PREDICTION MODEL GENERATION DEVICE, AND PROGRAM FOR COGNITIVE IMPAIRMENT PREDICTION**

VORRICHTUNG ZUR VORHERSAGE VON KOGNITIVEN STÖRUNGEN, VORRICHTUNG ZUR ERZEUGUNG EINES VORHERSAGEMODELLS UND PROGRAMM ZUR VORHERSAGE VON KOGNITIVEN STÖRUNGEN

DISPOSITIF DE PRÉDICTION DE DÉFICIENCE COGNITIVE, DISPOSITIF DE GÉNÉRATION DE MODÈLES DE PRÉDICTION, ET PROGRAMME DE PRÉDICTION DE DÉFICIENCE COGNITIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.09.2018 JP 2018170847**

(43) Date of publication of application:
**16.06.2021 Bulletin 2021/24**

(73) Proprietors:
 • **FRONTEO, Inc.**
  **Tokyo 108-0075 (JP)**
 • **Keio University**
  **Tokyo 108-8345 (JP)**

(72) Inventors:
 • **TOYOSHIBA, Hiroyoshi**
  **Tokyo 108-0075 (JP)**
 • **UCHIYAMA, Hidefumi**
  **Tokyo 108-0075 (JP)**
 • **KISHIMOTO, Taishiro**
  **Tokyo 160-8582 (JP)**
 • **FUNAKI, Kei**
  **Tokyo 160-8582 (JP)**
 • **SUGA, Yoko**
  **Tokyo 160-8582 (JP)**
 • **HOTTA, Shogo**
  **Tokyo 160-8582 (JP)**
 • **FUJITA, Takanori**
  **Tokyo 160-8582 (JP)**
 • **MIMURA, Masaru**
  **Tokyo 160-8582 (JP)**

(74) Representative: **Vossius & Partner
Patentanwälte Rechtsanwälte mbB
Siebertstrasse 3
81675 München (DE)**

(56) References cited:
JP-A- 2005 208 782    JP-A- 2018 015 139
JP-A- 2018 032 213

- **SWETA KARLEKAR ET AL: "Detecting Linguistic Characteristics of Alzheimer's Dementia by Interpreting Neural Models", ARXIV.ORG, CORNELL UNIVERSITY LIBRARY, 201 OLIN LIBRARY CORNELL UNIVERSITY ITHACA, NY 14853, 17 April 2018 (2018-04-17), XP080871826,**
- **SHIBATA, DAISAKU et al.: "Pronoun Increases in Alzheimer's Disease", Information Processing Society of Japan, vol. 2016, no. 13, 28 July 2016 (2016-07-28), pages 1-7, XP055787165,**

**Description**

Technical Field

The present invention relates to a dementia prediction device, a prediction model generation device, and a dementia prediction program, and particularly relates to a technology for predicting the severity of dementia of a patient (including a possibility that the patient has dementia), and a technology for generating a prediction model used for this prediction.

Background Art

Dementia continues to increase with the aging of the population, and has become a major social issue as well as a medical problem. Early detection and evaluation of severity of dementia are significantly important in the treatment of dementia. Currently, the mini-mental state examination (MMSE) has been widely used in daily clinical practice for screening tests for dementia and evaluation of severity. The MMSE is a cognitive function test including 11 items and 30 points of questions for examining insight, memory, attention (calculation), linguistic ability, composition ability (graphical ability), etc. Of the 30 points, 27 points or less is suspected of mild cognitive impairment (MCI), and 23 points or less is suspected of dementia.

Conventionally, there has been known a system in which evaluation is performed for each evaluation item of the MMSE to determine a possibility of developing dementia, and nursing care support is provided based on a determination result (for example, see Patent Document 1). In the system described in Patent Document 1, a physical or mental health condition of a care recipient is investigated by MMSE, and the health condition of the care recipient is evaluated from an investigation result. Then, audio or video is created according to the evaluation of the health condition of the care recipient and distributed to a caregiver, and the caregiver cares for the care recipient based on the distributed audio or video. Thereafter, the physical or mental health condition of the care recipient is re-examined and the health condition of the care recipient is re-investigated. It is stated that the investigation is conducted from the perspectives of four items of memory impairment, insight, activities of daily living (ADL), and physical function.

Citation List

Patent Document

Patent Document 1: JP-A-2002-251467

Non-patent document: Sweta Karlekar et al., "Detecting Linguistic Characteristics of Alzheimer's Dementia by Interpreting Neural Models", https://arxiv.org/abs/1804.06440, 17-04-2018.

Summary of the Invention

Technical Problem

The MMSE has been widely known as a highly reproducible test. However, when the same patient is tested a plurality of times, a practice effect causes the patient to memorize the content of the question, making it impossible to measure an accurate score. Therefore, there is a problem that it is difficult to frequently measure the severity of dementia. The system described in Patent Document 1 described above does not take into consideration the problem that the MMSE is unsuitable for repeated use.

The invention has been made to solve such a problem, and an object of the invention is to obtain a measurement result excluding a practice effect by a patient even when the severity of dementia is repeatedly measured.

Solution to Problem

The scope of the invention is determined by the appended independent claims. Preferable embodiments are determined by the appended dependent claims.

Advantageous Effects of the Invention

According to the invention configured as described above, since severity of dementia is predicted by analyzing a free conversation conducted by a patient, there is no need to perform a mini-mental state examination (MMSE) . For this reason, even when the severity of the dementia is repeatedly measured, it is possible to obtain a measurement result (prediction result) excluding a practice effect by a patient. In particular, when a patient suffers from dementia, a conversational characteristic peculiar to dementia can be seen in a free conversation, a relationship index value is computed in a state where such a conversational characteristic is reflected, and a prediction model is generated using the relationship index value. Thus, it is possible to predict severity of the dementia from the free conversation conducted by the patient.

Brief Description of the Drawings

Fig. 1 is a block diagram illustrating a functional configuration example of a dementia prediction device according to a first embodiment.
Fig. 2 is an explanatory diagram of a text index value group according to the first embodiment.
Fig. 3 is a flowchart illustrating an operation example of the dementia prediction device according to the first embodiment.
Fig. 4 is a block diagram illustrating a functional configuration example of a dementia prediction device according to a second embodiment.
Fig. 5 is a diagram illustrating processing content of a part-of-speech extraction unit according to the second embodiment.
Fig. 6 is a diagram showing an example of a part of speech extracted by the part-of-speech extraction unit according to the second embodiment.
Fig. 7 is a block diagram illustrating a functional configuration example of a dementia prediction device according to a third embodiment.
Fig. 8 is a diagram illustrating processing content of a prediction model generation unit according to the third embodiment.
Fig. 9 is a block diagram illustrating a functional configuration example of a dementia prediction device according to a fourth embodiment.
Fig. 10A and 10B are block diagrams illustrating a functional configuration example of the dementia prediction device according to the fourth embodiment.
Fig. 11 is a block diagram illustrating a functional configuration example of a dementia prediction device according to a fifth embodiment.
Fig. 12 is a block diagram illustrating a modification of the dementia prediction device.

Mode for Carrying Out the Invention

(First embodiment)

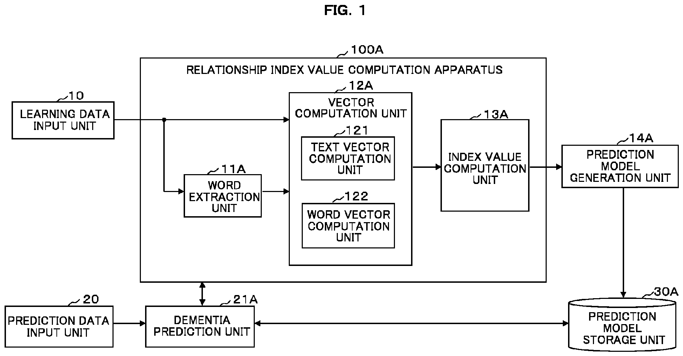

Hereinafter, a first embodiment according to the invention will be described with reference to the drawings. Fig. 1 is a block diagram illustrating a functional configuration example of a dementia prediction device according to the first embodiment. The dementia prediction device according to the first embodiment includes a learning data input unit 10, a word extraction unit 11A, a vector computation unit 12A, an index value computation unit 13A, a prediction model generation unit 14A, a prediction data input unit 20, and a dementia prediction unit 21A as a functional configuration. The vector computation unit 12A includes a text vector computation unit 121 and a word vector computation unit 122 as a more specific functional configuration. In addition, the dementia prediction device of the present embodiment includes a prediction model storage unit 30A as a storage medium. Note that for convenience of explanation, a part including the word extraction unit 11A, the vector computation unit 12A, and the index value computation unit 13A is referred to as a relationship index value computation unit 100A.

The relationship index value computation unit 100A inputs text data related to a text, and computes and outputs a relationship index value reflecting a relationship between the text and a word contained therein. In addition, in the dementia prediction device of the present embodiment, the relationship index value computation unit 100A analyzes a text representing content of a free conversation conducted by a patient, and severity of dementia of the patient is predicted from the content of the free conversation by the patient using a relationship index value computed by the analysis. A prediction model generation device of the invention includes the learning data input unit 10, the relationship index value computation unit 100A, and the prediction model generation unit 14A.

In the present specification, the term "text" generally refers to a text including two or more sentences divided by a period. In particular, in the present specification, a plurality of remark contents (corresponding to a plurality of sentences) spoken by a patient in a series of free conversations (continuous dialogue) conducted between a doctor and the patient is collectively treated as one text. That is, one text including a plurality of sentences is defined for one free conversation (a series of dialogues) of one patient.

Each of the functional blocks illustrated in Fig. 1 can be configured by any of hardware, a Digital Signal Processor (DSP), and software. For example, in the case of being configured by software, each of the functional blocks actually includes a CPU, a RAM, a ROM, etc. of a computer, and is implemented by operation of a program stored in a recording medium such as a RAM, a ROM, a hard disk, or a semiconductor memory.

The learning data input unit 10 inputs, as learning data, m texts representing contents of free conversations conducted by m patients (m is an arbitrary integer of 2 or more) whose severity of dementia is known, respectively. For example, the learning data input unit 10 replaces voice of a free conversation conducted between a patient and a doctor given an MMSE score by a pre-trained doctor with character data, and inputs a text of an utterance part of the patient included in the character data as learning data. In this case, the known severity of dementia for the patient means a value of the MMSE score. The learning data input unit 10 inputs m texts acquired from free conversations of m patients, respectively, as a plurality of pieces of learning data.

For example, the free conversation between the patient and the doctor is conducted in the form of an interview for 5 to 10 minutes. That is, a dialogue in the form in which the doctor asks the patient a question, and the patient answers the question is repeatedly conducted. Then, the dialogue at this time is input from a microphone and recorded, and voice of a series of dialogues (free conversations) is replaced with character data by manual transcription or using automatic voice recognition technology. From this character data, only the utterance part by the patient is extracted and used as learning data. Note that when the voice of the free conversation is replaced with the character data, only the utterance part by the patient may be replaced with the character data.

The word extraction unit 11A is an example of an "element extraction unit" in the claims, which analyzes m texts input as learning data by the learning data input unit 10, and extracts n words (n is an arbitrary integer of 2 or more) (corresponding to a decomposition element in the claims) from the m texts. As a method of analyzing texts, for example, a known morphological analysis can be used. Here, the word extraction unit 11A may extract morphemes of all parts of speech divided by the morphological analysis as words, or may extract only morphemes of a specific part of speech as words.

Note that the same word may be included in the m texts a plurality of times. In this case, the word extraction unit 11A does not extract the plurality of the same words, and extracts only one. That is, the n words extracted by the word extraction unit 11A refer to n types of words. However, each of the extracted n words is accompanied by information indicating an appearance frequency in the text. Here, the word extraction unit 11A may measure a frequency at which the same word is extracted from the m texts, and extract n (n types of) words from the one having the highest appearance frequency, or n (n types of) words having the appearance frequency equal to or higher than a threshold value.

A patient suffering from dementia has a tendency to repeat words spoken by the patient once many times . In addition, the patient suffering from dementia is less likely to speak spontaneously and may have a tendency to repeat conversations (echolalia) in which similar words are repeated in response to questions from doctors. Therefore, the word extraction unit 11A extracts n words from a text of a free conversation including a conversational characteristic peculiar to dementia.

The vector computation unit 12A computes m text vectors and n word vectors from the m texts and the n words. Here, the text vector computation unit 121 converts each of the m texts to be analyzed by the word extraction unit 11A into a q-dimensional vector (q is an arbitrary integer of 2 or more) according to a predetermined rule, thereby computing the m text vectors including q axis components. In addition, the word vector computation unit 122 converts each of the n words extracted by the word extraction unit 11A into a q-dimensional vector according to a predetermined rule, thereby computing the n word vectors including q axis components.

In the present embodiment, as an example, a text vector and a word vector are computed as follows. Now, a set S = <d ∈ D, w ∈ W> including the m texts and the n words is considered. Here, a text vector $\vec{d_i}$ and a word vector $\vec{w_j}$ (hereinafter, the symbol "→" indicates a vector) are associated with each text $d_i$ (i = 1, 2, ..., m) and each word $w_j$ (j = 1, 2, ..., n), respectively. Then, a probability $P(w_j|d_i)$ shown in the following Equation (1) is calculated with respect to an arbitrary word $w_j$ and an arbitrary text $d_i$.

[Equation 1]

$$P(w_j \mid d_i) = \frac{\exp(\vec{w_j} \cdot \vec{d_i})}{\sum_{k=1}^{n} \exp(\vec{w_k} \cdot \vec{d_i})} \quad \cdots (1)$$

Note that the probability $P(w_j|d_i)$ is a value that can be computed in accordance with a probability p disclosed in, for example, a follow thesis describing evaluation of a text or a document by a paragraph vector. "'Distributed Representations of Sentences and Documents' by Quoc Le and Tomas Mikolov, Google Inc; Proceedings of the 31st International Conference on Machine Learning Held in Bejing, China on 22-24 June 2014" This thesis states that, for example, when there are three words "the", "cat", and "sat", "on" is predicted as a fourth word, and a computation formula of the prediction probability p is described. The probability p(wt|wt - k, ..., wt + k) described in the thesis is a correct answer probability when another word wt is predicted from a plurality of words wt - k, ..., wt + k.

Meanwhile, the probability $P(w_j|d_i)$ shown in Equation (1) used in the present embodiment represents a correct answer probability that one word $w_j$ of n words is predicted from one text $d_i$ of m texts. Predicting one word $w_j$ from one text $d_i$ means that, specifically, when a certain text $d_i$ appears, a possibility of including the word $w_j$ in the text $d_i$ is predicted.

In Equation (1), an exponential function value is used, where e is the base and the inner product of the word vector $w\rightarrow$ and the text vector $d\rightarrow$ is the exponent. Then, a ratio of an exponential function value calculated from a combination of a text $d_i$ and a word $w_j$ to be predicted to the sum of n exponential function values calculated from each combination of the text $d_i$ and n words $w_k$ (k = 1, 2, ..., n) is calculated as a correct answer probability that one word $w_j$ is expected from one text $d_i$.

Here, the inner product value of the word vector $w_j\rightarrow$ and the text vector $d_i\rightarrow$ can be regarded as a scalar value when the word vector $w_j\rightarrow$ is projected in a direction of the text vector $d_i\rightarrow$, that is, a component value in the direction of the text vector $d_i\rightarrow$ included in the word vector $w_j\rightarrow$, which can be considered to represent a degree at which the word $w_j$ contributes to the text $d_i$. Therefore, obtaining the ratio of the exponential function value calculated for one word $w_j$ to the sum of the exponential function values calculated for n words $w_k$ (k = 1, 2, ..., n) using the exponential function value calculated using the inner product corresponds to obtaining the correct answer probability that one word $w_j$ of n words is predicted from one text $d_i$.

Note that since Equation (1) is symmetrical with respect to $d_i$ and $w_j$, a probability $P(d_i|w_j)$ that one text $d_i$ of m texts is predicted from one word $w_j$ of n words may be calculated. Predicting one text $d_i$ from one word $w_j$ means that, when a certain word $w_j$ appears, a possibility of including the word $w_j$ in the text $d_i$ is predicted. In this case, an inner product value of the text vector $d_i\rightarrow$ and the word vector $w_j\rightarrow$ can be regarded as a scalar value when the text vector $d_i\rightarrow$ is projected in a direction of the word vector $w_j\rightarrow$, that is, a component value in the direction of the word vector $w_j\rightarrow$ included in the text vector $d_i\rightarrow$, which can be considered to represent a degree at which the text $d_i$ contributes to the word $w_j$.

Note that here, a calculation example using the exponential function value using the inner product value of the word vector $w\rightarrow$ and the text vector $d\rightarrow$ as an exponent has been described. However, the exponential function value may not be used. Any calculation formula using the inner product value of the word vector $w\rightarrow$ and the text vector $d\rightarrow$ may be used. For example, the probability may be obtained from the ratio of the inner product values itself (however, including performing a predetermined operation for obtaining a positive value as the inner product value at all times (for example, inner product value + 1)).

Next, the vector computation unit 12A computes the text vector $d_i\rightarrow$ and the word vector $w_j\rightarrow$ that maximize a value L of the sum of the probability $P(w_j|d_i)$ computed by Equation (1) for all the set S as shown in the following Equation (2). That is, the text vector computation unit 121 and the word vector computation unit 122 compute the probability $P(w_j|d_i)$ computed by Equation (1) for all combinations of the m texts and the n words, and compute the text vector $d_i\rightarrow$ and the word vector $w_j\rightarrow$ that maximize a target variable L using the sum thereof as the target variable L.

[Equation 2]

$$L = \sum_{d \in D} \sum_{w \in W} \#(w, d)\, p(w \mid d) \quad \cdots (2)$$

Maximizing the total value L of the probability $P(w_j|d_i)$ computed for all the combinations of the m texts and the n words corresponds to maximizing the correct answer probability that a certain word $w_j$ (j = 1, 2, ..., n) is predicted from a certain text $d_i$ (i = 1, 2, ..., m) . That is, the vector computation unit 12A can be considered to compute the text vector $d_i\rightarrow$ and the word vector $w_j\rightarrow$ that maximize the correct answer probability.

Here, in the present embodiment, as described above, the vector computation unit 12A converts each of the m texts $d_i$ into a q-dimensional vector to compute the m texts vectors $d_i\rightarrow$ including the q axis components, and converts each of the n words into a q-dimensional vector to compute the n word vectors $w_j\rightarrow$ including the q axis components, which corresponds to computing the text vector $d_i\rightarrow$ and the word vector $w_j\rightarrow$ that maximize the target variable L by making q axis directions variable.

The index value computation unit 13A takes each of the inner products of the m text vectors $d_i\rightarrow$ and the n word vectors $w_j\rightarrow$ computed by the vector computation unit 12A, thereby computing m × n relationship index values reflecting the relationship between the m texts $d_i$ and the n words $w_j$. In the present embodiment, as shown in the following Equation (3), the index value computation unit 13A obtains the product of a text matrix D having the respective q axis components ($d_{11}$ to $d_{mq}$) of the m text vectors $d_i\rightarrow$ as respective elements and a word matrix W having the respective q axis components ($w_{11}$ to $w_{nq}$) of the n word vectors $w_j\rightarrow$ as respective elements, thereby computing an index value matrix DW having m × n relationship index values as elements. Here, $W^t$ is the transposed matrix of the word matrix.

[Equation 3]

$$D = \begin{bmatrix} d_{11} & d_{12} & \cdots & d_{1q} \\ d_{21} & d_{22} & \cdots & d_{2q} \\ \cdot & \cdot & \cdot & \cdot \\ \cdot & \cdot & \cdot & \cdot \\ \cdot & \cdot & \cdot & \cdot \\ d_{m1} & d_{m2} & \cdots & d_{mq} \end{bmatrix} \qquad W = \begin{bmatrix} w_{11} & w_{12} & \cdots & w_{1q} \\ w_{21} & w_{22} & \cdots & w_{2q} \\ \cdot & \cdot & \cdot & \cdot \\ \cdot & \cdot & \cdot & \cdot \\ \cdot & \cdot & \cdot & \cdot \\ w_{n1} & w_{m2} & \cdots & w_{mq} \end{bmatrix}$$

$$DW = D * W^t = \begin{bmatrix} dw_{11} & dw_{12} & \cdots & dw_{1n} \\ dw_{21} & dw_{22} & \cdots & dw_{2n} \\ \cdot & \cdot & \cdot & \cdot \\ \cdot & \cdot & \cdot & \cdot \\ \cdot & \cdot & \cdot & \cdot \\ dw_{m1} & dw_{m2} & \cdots & dw_{mn} \end{bmatrix} \qquad \cdots (3)$$

Each element $dw_{ij}$ (i = 1, 2, ..., m; j = 1, 2, ..., n) of the index value matrix DW computed in this manner may indicate which word contributes to which text and to what extent. For example, an element $dw_{12}$ in the first row and the second column is a value indicating a degree at which the word $w_2$ contributes to a text $d_1$. In this way, each row of the index value matrix DW can be used to evaluate the similarity of a text, and each column can be used to evaluate the similarity of a word.

The prediction model generation unit 14A generates a prediction model for predicting severity of dementia based on a text index value group including n relationship index values $dw_{ij}$ (j = 1, 2, ..., n) for one text $d_i$ using m × n relationship index values computed by the index value computation unit 13A. Here, the severity of the dementia to be predicted is a value of an MMSE score. That is, the prediction model generation unit 14A generates a prediction model in which a score as close to x points as possible is predicted for a text index value group computed based on a free conversation of a patient whose MMSE score is known (for example, x points). Then, the prediction model generation unit 14A causes the prediction model storage unit 30A to store the generated prediction model.

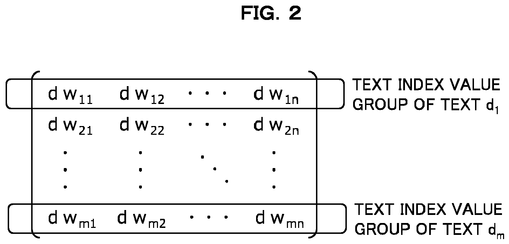

Fig. 2 is a diagram for description of a text index value group. As illustrated in Fig. 2, for example, in the case of the first text $d_1$, n relationship index values $dw_{11}$ to $dw_{1n}$ included in a first row of an index value matrix DW correspond to a text index value group. Similarly, in the case of the second text $d_2$, n relationship index values $dw_{21}$ to $dw_{2n}$ included in a second row of the index value matrix DW correspond to a text index value group. Hereinafter, this description is similarly applied up to a text index value group related to an mth text $d_m$ (n relationship index values $dw_{m1}$ to $dw_{mn}$).

The prediction model generation unit 14A computes each feature quantity associated with severity of dementia for a text index value group of each of the texts $d_i$ (i = 1, 2, ..., m) using m × n relationship index values $dw_{11}$ to $dw_{mn}$ computed by the index value computation unit 13A, and generates a prediction model for predicting severity of dementia from one text index value group based on the computed feature quantity. Here, the prediction model generated by the prediction model generation unit 14A is a learning model in which a text index value group of a text $d_i$ is input and an MMSE score is output as a solution.

For example, a form of the prediction model generated by the prediction model generation unit 14A may be set to any one of a regression model (learning model based on linear regression, logistic regression, support vector machine, etc.), a tree model (learning model based on decision tree, regression tree, random forest, gradient boosting tree, etc.), a neural network model (learning model based on perceptron, convolutional neural network, recurrent neural network, residual network, RBF network, stochastic neural network, spiking neural network, complex neural network, etc.), a Bayesian model (learning model based on Bayesian inference), a clustering model (learning model based on k-nearest neighbor method, hierarchical clustering, non-hierarchical clustering, topic model, etc.), etc. Note that the prediction models listed here are merely examples, and the invention is not limited thereto.

The feature quantity computed when the prediction model generation unit 14A generates the prediction model may be computed by a predetermined algorithm. In other words, a method of computing the feature quantity performed by the prediction model generation unit 14A may be arbitrarily designed. For example, the prediction model generation unit 14A performs predetermined weighting calculation on each text index value group of each text $d_i$ so that a value obtained by the weighting calculation approaches a known value (MMSE score) indicating the severity of dementia, and generates a prediction model for predicting the severity of dementia (MMSE score) from the text index value group of the text $d_i$ using a weighted value for the text index value group as the feature quantity.

In more detail, with regard to the text index value group of the first text $d_i$ including n relationship index values

$dw_{11}$ to $dw_{1n}$ contained in the first row of the index value matrix DW, a weighted value $\{a_{11}, a_{12}, ..., a_{1n}\}$ is computed as a feature quantity so that the following expression is satisfied.

$$a_{11} \cdot dw_{11} + a_{12} \cdot dw_{12} + ... \ a_{1n} \cdot dw_{1n} \approx known \ score \ for \ MMSE$$

In addition, with regard to the text index value group of the second text $d_2$ including n relationship index values $dw_{21}$ to $dw_{2n}$ contained in the second row of the index value matrix DW, a weighted value $\{a_{21}, a_{22}, ..., a_{2n}\}$ is computed as a feature quantity so that the following expression is satisfied.

$$a_{21} \cdot dw_{21} + a_{22} \cdot dw_{22} + ... \ a_{2n} \cdot dw_{2n} \approx known \ score \ for \ MMSE$$

Similarly, with regard to the text index value group of the mth text $d_m$, a weighted value $\{a_{m1}, a_{m2}, ..., a_{mn}\}$ is computed as a feature quantity so that the following expression is satisfied.

$$a_{m1} \cdot dw_{m1} + a_{m2} \cdot dw_{m2} + ... \ a_{mn} \cdot dw_{mn} \approx known \ score \ for \ MMSE$$

Then, a prediction model in which each of these feature quantities is associated with a known score for the MMSE is generated.

Note that, here, a description has been given of an example in which each of m sets of weighted values $\{a_{11}, a_{12}, ..., a_{1n}\}, ..., \{a_{m1}, a_{m2}, ..., a_{mn}\}$ are used as feature quantities. However, the invention is not limited thereto. For example, using text index value groups obtained from learning data of patients having the same score for the MMSE among m text index value groups obtained from m pieces of learning data related to the m patients, one or a plurality of weight values having a characteristic common to these text index value or predetermined calculated values using the plurality of weight values, etc. may be extracted as a feature quantity.

The prediction data input unit 20 inputs, as prediction data, m' texts representing contents of free conversations conducted by m' patients (m' is an arbitrary integer of 1 or more) subjected to prediction, respectively. That is, the prediction data input unit 20 replaces voice of a free conversation between a doctor and a patient whose score for the MMSE is unknown with character data, and inputs a text of an utterance part of the patient included in the character data as prediction data. A method of acquiring m' texts from a free conversation between a doctor and a patient subjected to prediction is similar to the method of acquiring m texts from a free conversation between a doctor and a patient to be learned.

The patient subjected to prediction may be a first-visit patient or a return-visit patient diagnosed with suspected dementia. When the first-visit patient is subjected to prediction, whether or not the patient is suspected of having dementia can be predicted and if the patient has dementia, the severity of the dementia can be predicted only by conducting a free conversation between the patient and the doctor by an interview without performing the MMSE on the patient as described below. Meanwhile, when the return-visit patient is subjected to prediction, the severity of dementia can be predicted only by conducting a free conversation between the patient and the doctor by an interview without performing the MMSE on the patient. In this way, it is possible to determine whether a symptom is ameliorating or worsening without being affected by the practice effect of the patient on the MMSE.

The dementia prediction unit 21A applies a relationship index value obtained by executing processes of the word extraction unit 11A, the text vector computation unit 121, the word vector computation unit 122, and the index value computation unit 13A on prediction data input by the prediction data input unit 20 to a prediction model generated by the prediction model generation unit 14A (prediction model stored in the prediction model storage unit 30A), thereby predicting severity of dementia for m' patients subjected to prediction.

For example, when m' texts acquired from free conversations of m' patients whose scores for the MMSE are unknown are input as prediction data by the prediction data input unit 20, m' text index value groups are obtained by executing a process of the relationship index value computation unit 100A for the m' texts according to an instruction of the dementia prediction unit 21A. The dementia prediction unit 21A assigns the m' text index value groups computed by the relationship index value computation unit 100A to the prediction model as input data, thereby predicting the severity of dementia related to each of the m' patients.

At the time of this prediction, the word extraction unit 11A extracts n words from the m' texts input by the prediction data input unit 20 as prediction data. The number of words extracted from the m' texts by the word extraction unit 11A during prediction is the same as the number n of words extracted from the m texts by the word extraction unit 11A during learning. Note that, for example, there is the case of m' = 1, that is, a case where n words are extracted from one text by a free conversation of one patient. Therefore, it is preferable to presume a standard type of word spoken by one patient in a free conversation in the form of an interview of about 5 to 10 minutes and determine a value of n so that a situation in which there is no overlap (same word) between n words extracted from one text of prediction data and n words extracted from m texts of learning data does not occur.

In addition, during prediction, the text vector computation unit 121 converts each of m' texts into a q-dimensional vector according to a predetermined rule, thereby computing m' text vectors including q axis components. The word vector computation unit 122 converts each of n words into a q-dimensional vector according to a predetermined rule, thereby computing n word vectors including q axis components. The index value computation unit 13A obtains each of inner products of the m' text vectors and the n word vectors, thereby computing m' × n relationship index values reflecting a relationship between the m' texts and the n words. The dementia prediction unit 21A applies m' × n relationship index values computed by the index value computation unit 13A to a prediction model stored in the prediction model storage unit 30A, thereby predicting severity of dementia for m' patients subjected to prediction.

Note that for the purpose of reducing an operation load during prediction, computation of a word vector by the word vector computation unit 122 may be omitted, and n word vectors computed during learning may be stored and used during prediction. In this way, a process of reading and using n word vectors computed during learning by the word vector computation unit 122 during prediction is included as one aspect of executing a process of the word vector computation unit 122 on the prediction data.

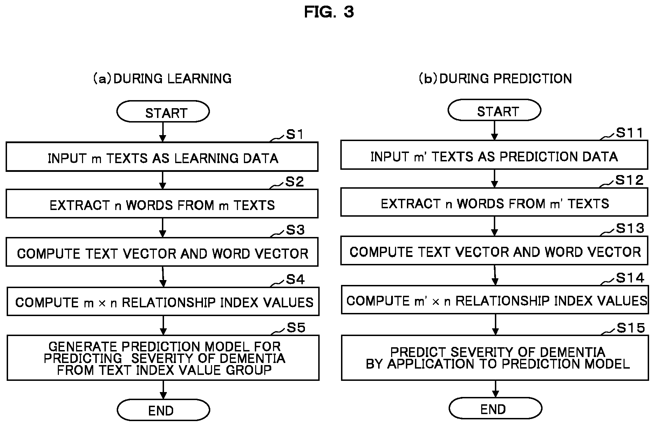

Fig. 3 is a flowchart illustrating an operation example of the dementia prediction device according to the first embodiment configured as described above. Fig. 3(a) illustrates an example of an operation during learning for generating a prediction model, and Fig. 3(b) illustrates an example of an operation during prediction for predicting severity of dementia using the generated prediction model.

During learning illustrated in Fig. 3(a), first, the learning data input unit 10 inputs, as learning data, m texts representing contents of free conversations conducted by m patients whose severity of dementia (score for the MMSE) is known, respectively (step S1). The word extraction unit 11A analyzes the m texts input by the learning data input unit 10, and extracts n words from the m texts (step S2).

Subsequently, the vector computation unit 12A computes m text vectors $d_i\rightarrow$ and n word vectors $w_j\rightarrow$ from the m texts input by the learning data input unit 10 and the n words extracted by the word extraction unit 11A (step S3). Then, the index value computation unit 13A obtains each of inner products of the m text vectors $d_i\rightarrow$ and the n word vectors $w_j\rightarrow$, thereby computing m × n relationship index values (index value matrix DW having m × n relationship index values as respective elements) reflecting a relationship between the m texts $d_i$ and the n words $w_j$ (step S4).

Further, as described above, the prediction model generation unit 14A generates a prediction model for predicting severity of dementia based on a text index value group including n relationship index values $dw_{ij}$ for one text $d_i$ using m × n relationship index values computed by the relationship index value computation unit 100A from learning data related to m patients, and causes the prediction model storage unit 30A to store the generated prediction model (step S5). In this way, an operation during learning is completed.

During prediction illustrated in Fig. 3(b), first, the prediction data input unit 20 inputs m' texts representing contents of free conversations conducted by m' patients subjected to prediction, respectively, as prediction data (step S11). The dementia prediction unit 21A supplies the prediction data input by the prediction data input unit 20 to the relationship index value computation unit 100A, and gives an instruction to compute a relationship index value.

In response to this instruction, the word extraction unit 11A analyzes the m' texts input by the prediction data input unit 20, and extracts n words from the m' texts (step S12). Subsequently, the vector computation unit 12A computes m' text vectors $d_i\rightarrow$ and n word vectors $w_j\rightarrow$ from the m' texts input by the prediction data input unit 20 and the n words extracted by the word extraction unit 11A (step S13).

Then, the index value computation unit 13A obtains each of inner products of the m' text vectors $d_i\rightarrow$ and the n word vectors $w_j\rightarrow$, thereby computing m' × n relationship index values (index value matrix DW having m' × n relationship index values as respective elements) reflecting a relationship between the m' texts $d_i$ and the n words $w_j$ (step S14). The index value computation unit 13A supplies the computed m' × n relationship index values to the dementia prediction unit 21A.

The dementia prediction unit 21A applies the m' × n relationship index values supplied from the relationship index value computation unit 100A to the prediction model stored in the prediction model storage unit 30A, thereby predicting severity of dementia for m' patients subjected to prediction (step S15). In this way, an operation during prediction is completed.

As described in detail above, in the first embodiment, m texts representing content of a free conversation conducted by a patient whose severity of dementia is known are input as learning data, an inner product of a text vector computed from the input texts and a word vector computed from words contained in the texts is calculated to compute a relationship index value reflecting a relationship between the texts and the words, and a prediction model is generated using this relationship index value. In addition, when severity of dementia is predicted for a patient subjected to prediction, m' texts representing content of a free conversation conducted by the patient subjected to prediction are input as prediction data, and a relationship index value similarly computed from the input prediction data is applied to a prediction model, thereby predicting the severity of dementia of the patient subjected to prediction.

According to the first embodiment configured as described above, it is unnecessary to perform a mini-mental state examination (MMSE) since the severity of dementia is predicted by analyzing a free conversation conducted by the patient. Therefore, even when the severity of dementia is repeatedly measured, it is possible to obtain a measurement result (prediction result) excluding the practice effect by the patient. In particular, when a patient suffers from dementia, a conversational characteristic peculiar to dementia, including words repeatedly spoken, can be seen in a free conversation, a relationship index value is computed in a state where such a conversational characteristic is reflected, and a prediction model is generated using the relationship index value. Thus, it is possible to predict the severity of dementia from the free conversation conducted by the patient.

(Second embodiment)

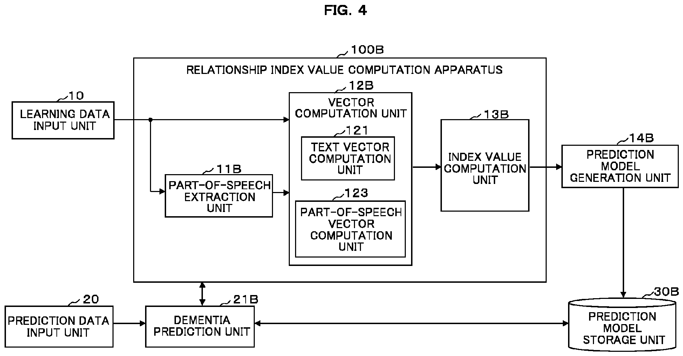

Next, a second embodiment according to the invention will be described with reference to the drawings. Fig. 4 is a block diagram illustrating a functional configuration example of a dementia prediction device according to the second embodiment. In Fig. 4, a component denoted by the same reference symbol as that illustrated in Fig. 1 has the same function, and thus duplicate description will be omitted here.

As illustrated in Fig. 4, the dementia prediction device according to the second embodiment includes a relationship index value computation unit 100B, a prediction model generation unit 14B, a dementia prediction unit 21B, and a prediction model storage unit 30B instead of the relationship index value computation unit 100A, the prediction model generation unit 14A, the dementia prediction unit 21A, and the prediction model storage unit 30A. The relationship index value computation unit 100B according to the second embodiment includes a part-of-speech extraction unit 11B, a vector computation unit 12B, and an index value computation unit 13B instead of the word extraction unit 11A, the vector computation unit 12A, and the index value computation unit 13A. The vector computation unit 12B includes a part-of-speech vector computation unit 123 instead of the word vector computation unit 122 as a more specific functional configuration. Note that a prediction model generation device of the invention includes the learning data input unit 10, the relationship index value computation unit 100B, and the prediction model generation unit 14B.

The relationship index value computation unit 100B according to the second embodiment inputs text data related to a text similar to that of the first embodiment, and computes and outputs a relationship index value reflecting a relationship between the text and a part of speech of each morpheme contained therein.

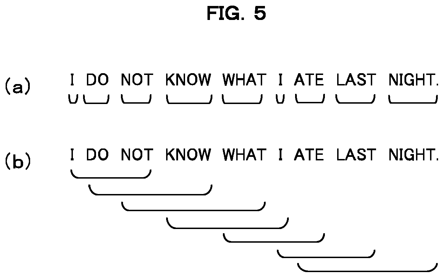

The part-of-speech extraction unit 11B is an example of an "element extraction unit" in the claims, which analyzes m texts input as learning data by the learning data input unit 10, and extracts p parts of speech (p is an arbitrary integer of 2 or more) (corresponding to decomposition elements in the claims) from the m texts. As a text analysis method, for example, it is possible to use a known morphological analysis. Here, for each morpheme divided by the morphological analysis, the part-of-speech extraction unit 11B may extract one part of speech for each single morpheme as illustrated in Fig. 5 (a) or extract one set of parts of speech for a plurality of consecutive morphemes as illustrated in Fig. 5(b).

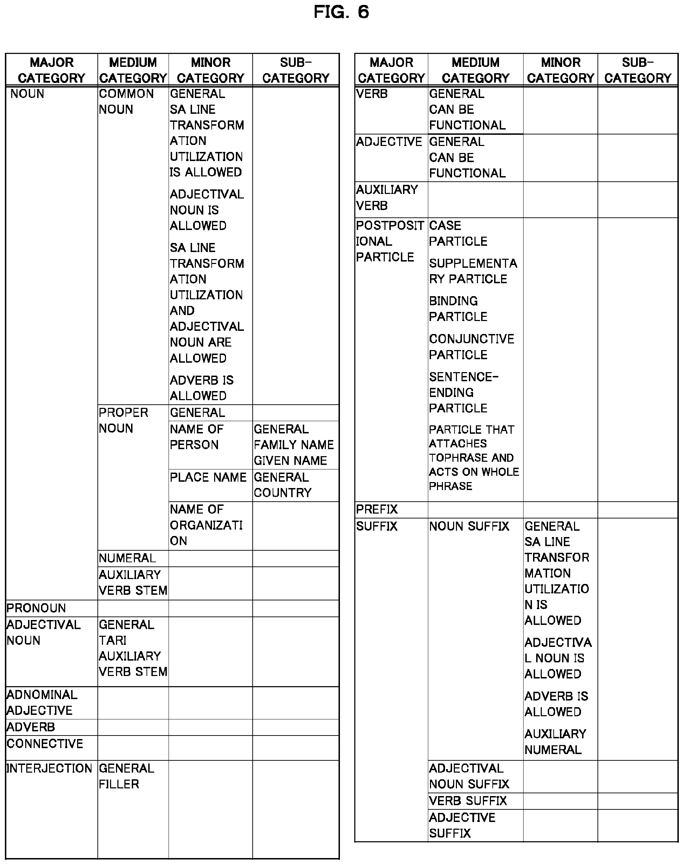

Note that in the present embodiment, as parts of speech to be extracted, parts of speech classified not only into major categories such as a verb, an adjective, an adjective verb, a noun, a pronoun, a numeral, an adnominal adjective, an adverb, a connective, an interjection, an auxiliary verb, and a postpositional particle, but also into a medium category, a minor category, and a sub-category as shown in Fig. 6 are extracted. Fig. 6 shows an example of parts of speech extracted by the part-of-speech extraction unit 11B. The parts of speech illustrated herein are an example, and the invention is not limited thereto.

Note that the same part of speech (or the same set of parts of speech) may be included in m texts a plurality of times. In this case, the part-of-speech extraction unit 11B does not extract the same part of speech (or the same set of parts of speech) the plurality of times, and extracts the same part of speech (or the same set of parts of speech) only once. In other words, p parts of speech (concept including p sets, which is similarly applied hereinafter) extracted by the part-of-speech extraction unit 11B refers to p types of parts of speech. However, each of the extracted p parts of speech is accompanied by information indicating an appearance frequency in the respective texts.

Patient suffering from dementia may not remember proper nouns and tend to frequently use demonstratives such as "that", "this", and "it". In addition, patients suffering from dementia may tend to frequently use fillers such as "well", "um", and "uh" without the following words coming out. For this reason, there is the same part of speech appearing many times in a text of a free conversation according to such a conversational characteristic peculiar to dementia. The part-of-speech extraction unit 11B extracts p parts of speech from the text of the free conversation having such a conversational characteristic peculiar to dementia.

The vector computation unit 12B computes m text vectors and p part-of-speech vectors from m texts and p parts of speech. Here, the text vector computation unit 121 converts each of m texts to be analyzed by the part-of-speech extraction unit 11B into a q-dimensional vector according to a predetermined rule, thereby computing m text vectors including q axis components. In addition, the part-of-speech vector computation unit 123 converts each of p parts of speech extracted by the part-of-speech extraction unit 11B into a q-dimensional vector according to a predetermined rule, thereby computing p part-of-speech vectors including q axis components.

A method of computing the text vectors and the part-of-speech vectors is similar to that of the first embodiment. That is, in the second embodiment, the vector computation unit 12B considers a set $S = <d \in D, h \in H>$ including m texts and p parts of speech. Here, a text vector $d_i\rightarrow$ and a part-of-speech vector $h_j\rightarrow$ are associated with each of texts $d_i$ (i = 1, 2, ..., m) and each of parts of speech $h_j$ (j = 1, 2, ..., p), respectively. Then, the vector computation unit 12B computes a probability $P(h_j|d_i)$ computed similarly to the above Equation (1) for all combinations of the m texts and the p parts of speech, sets a total value as a target variable L, and computes a text vector $d_i\rightarrow$ and a part-of-speech vector $h_j\rightarrow$ that maximize the target variable L.

The index value computation unit 13B obtains each of inner products of m text vectors $d_i\rightarrow$ and p part-of-speech vectors $h_j\rightarrow$ computed by the vector computation unit 12B, thereby computing m × p relationship index values reflecting a relationship between m texts $d_i$ and p parts of speech $h_j$. In the second embodiment, as shown in the following Equation (4), the index value computation unit 13B obtains a product of a text matrix D having q respective axis components ($d_{11}$ to $d_{mq}$) of the m text vectors $d_i\rightarrow$ as respective elements and a part-of-speech matrix H having q respective axis components ($h_{11}$ to $h_{pq}$) of the p part-of-speech vectors $h_j\rightarrow$ as respective elements, thereby computing an index value matrix DH having the m × p relationship index values as respective elements. Here, $H^t$ is a transposed matrix of the part-of-speech matrix.

[Equation 4]

$$ D = \begin{bmatrix} d_{11} & d_{12} & \cdots & d_{1q} \\ d_{21} & d_{22} & \cdots & d_{2q} \\ \cdot & \cdot & \cdot & \cdot \\ \cdot & \cdot & \cdot & \cdot \\ \cdot & \cdot & \cdot & \cdot \\ d_{m1} & d_{m2} & \cdots & d_{mq} \end{bmatrix} \qquad H = \begin{bmatrix} h_{11} & h_{12} & \cdots & h_{1q} \\ h_{21} & h_{22} & \cdots & h_{2q} \\ \cdot & \cdot & \cdot & \cdot \\ \cdot & \cdot & \cdot & \cdot \\ \cdot & \cdot & \cdot & \cdot \\ h_{p1} & h_{p2} & \cdots & h_{pq} \end{bmatrix} $$

$$ DH = D * H^t = \begin{bmatrix} dh_{11} & dh_{12} & \cdots & dh_{1p} \\ dh_{21} & dh_{22} & \cdots & dh_{2p} \\ \cdot & \cdot & \cdot & \cdot \\ \cdot & \cdot & \cdot & \cdot \\ \cdot & \cdot & \cdot & \cdot \\ dh_{m1} & dh_{m2} & \cdots & dh_{mp} \end{bmatrix} \qquad \cdots (4) $$

The prediction model generation unit 14B generates a prediction model for predicting severity of dementia (score value for the MMSE) based on a text index value group including p relationship index values $dh_{ij}$ (j = 1, 2, ..., p) for one text $d_i$ using the m × p relationship index values computed by the index value computation unit 13B. That is, using a similar method to that described in the first embodiment, the prediction model generation unit 14B generates a prediction model in which a score as close to x points as possible is predicted for a text index value group computed based on a free conversation of a patient whose score for the MMSE is known (for example, x points). Then, the prediction model generation unit 14B causes the prediction model storage unit 30B to store the generated prediction model.

The dementia prediction unit 21B applies a relationship index value obtained by executing processes of the part-of-speech extraction unit 11B, the text vector computation unit 121, the part-of-speech vector computation unit 123, and the index value computation unit 13B on prediction data input by the prediction data input unit 20 to a prediction model generated by the prediction model generation unit 14B (prediction model stored in the prediction model storage unit 30B), thereby predicting severity of dementia for m' patients subjected to prediction.

As described in detail above, in the second embodiment, m texts representing content of a free conversation conducted by a patient whose severity of dementia is known are input as learning data, an inner product of a text vector computed from the input texts and a part-of-speech vector computed from a part of speech of a morpheme contained in the text is calculated, thereby computing a relationship index value reflecting a relationship between the text and the part of speech, and a prediction model is generated using this relationship index value. In addition, when severity of dementia is predicted for a patient subjected to prediction, m' texts representing content of a free conversation conducted by the patient subjected to prediction are input as prediction data, and a relationship index value similarly computed from the input prediction data is applied to a prediction model, thereby predicting the severity of dementia of the patient subjected to prediction.

Also in the second embodiment configured in this way, since the severity of dementia is predicted by analyzing the free conversation conducted by the patient, it is unnecessary to perform the mini-mental state examination (MMSE) . For this reason, even when the severity of dementia is repeatedly measured, it is possible to obtain a measurement result (prediction result) excluding the practice effect by the patient. In particular, when a patient suffers from dementia, a conversational characteristic peculiar to dementia containing a lot of morphemes of a predetermined part of speech can be seen in a free conversation, a relationship index value is computed in a state where such a conversational characteristic is reflected, and a prediction model is generated using the relationship index value. Thus, it is possible to predict the severity of dementia from the free conversation conducted by the patient.

(Third embodiment)

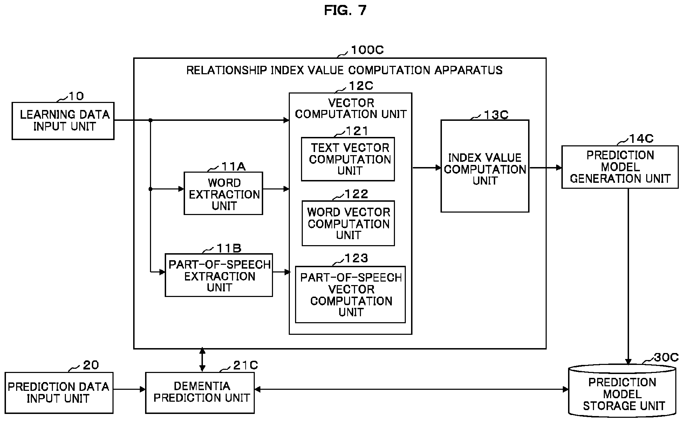

Next, a third embodiment according to the invention will be described with reference to the drawings. Fig. 7 is a block diagram illustrating a functional configuration example of a dementia prediction device according to the third embodiment. In Fig. 7, a component denoted by the same reference symbol as that illustrated in Fig. 4 has the same function, and thus duplicate description will be omitted here. The third embodiment uses both the index value matrix DW computed from the text vector and the word vector described in the first embodiment and the index value matrix DH computed from the text vector and the part-of-speech vector described in the second embodiment.

As illustrated in Fig. 7, the dementia prediction device according to the third embodiment includes a relationship index value computation unit 100C, a prediction model generation unit 14C, a dementia prediction unit 21C, and a prediction model storage unit 30C instead of the relationship index value computation unit 100B, the prediction model generation unit 14B, the dementia prediction unit 21B, and the prediction model storage unit 30B. The relationship index value computation unit 100C according to the third embodiment includes the word extraction unit 11A and the part-of-speech extraction unit 11B, and includes a vector computation unit 12C and an index value computation unit 13C instead of the vector computation unit 12B and the index value computation unit 13B. As a more specific functional configuration, the vector computation unit 12C includes a text vector computation unit 121, a word vector computation unit 122, and a part-of-speech vector computation unit 123. Note that a prediction model generation device of the invention includes the learning data input unit 10, the relationship index value computation unit 100C, and the prediction model generation unit 14C.

As shown in the above Equation (3), the index value computation unit 13C obtains each of inner products of m text vectors $d_i\rightarrow$ and n word vectors $w_j\rightarrow$, thereby computing $m \times n$ relationship index values $dw_{ij}$ (referred to as a first index value matrix DW) reflecting a relationship between m texts $d_i$ and n words $w_j$. In addition, as shown in the above Equation (4), the index value computation unit 13C obtains each of inner products of m text vectors $d_i\rightarrow$ and p part-of-speech vectors $h_j\rightarrow$, thereby computing $m \times p$ relationship index values $dh_{ij}$ (referred to as a second index value matrix DH) reflecting a relationship between m texts $d_i$ and p parts of speech $h_j$.

The prediction model generation unit 14C generates a prediction model for predicting severity of dementia (score value for the MMSE) based on a text index value group $dw_{ij}$ (j = 1, 2, ..., n) including n relationship index values and a text index value group $dh_{ij}$ (j = 1, 2, ..., p) including p relationship index values for one text $d_i$ using $m \times n$ relationship index values $dw_{ij}$ and $m \times p$ relationship index values $dh_{ij}$ computed by the index value computation unit 13C. Then, the prediction model generation unit 14C causes the prediction model storage unit 30C to store the generated prediction model.

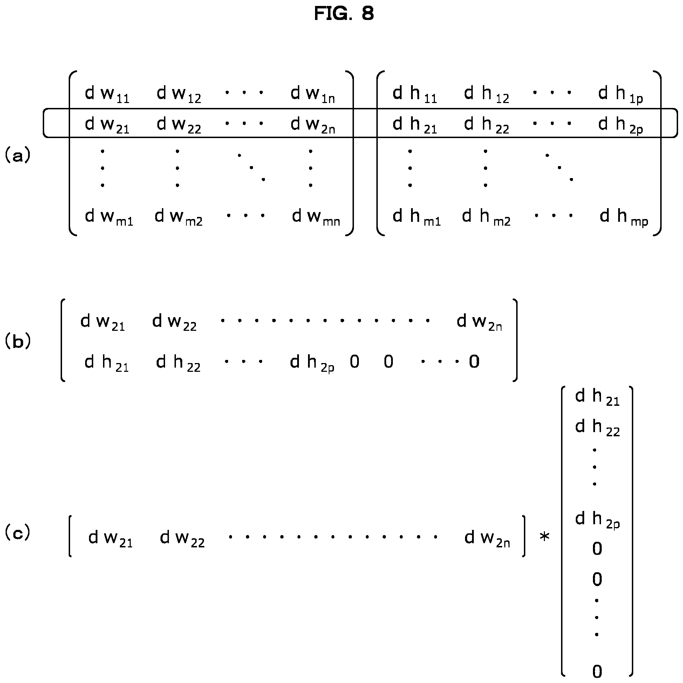

Here, it is possible to arbitrarily design a scheme in which the prediction model generation unit 14C uses two sets of text index value groups $dw_{ij}$ and $dh_{ij}$ to generate a prediction model. For example, as illustrated in Fig. 8(a), the first index value matrix DW between texts/words and the second index value matrix DH between texts/parts of speech may be arranged horizontally (row direction), text index value groups $dw_{ij}$ and $dh_{ij}$ belonging to the same row i may be connected to generate one text index value group including (n + p) relationship index values, and a prediction model for predicting severity of dementia may be generated based on this text index value group.

Alternatively, as illustrated in Fig. 8 (b), a text index value group $dw_{ij}$ on an ith row included in the first index value matrix DW between texts/words and a text index value group $dh_{ij}$ on the same ith row included in the second index value matrix DH between texts/parts of speech may be arranged vertically (column direction) to generate a $(2 \times n)$ -dimensional text index value group matrix, and a prediction model for predicting severity of dementia may be generated based on this text index value group matrix. In the example of Fig. 8(b), n > p is presumed, values of the text index value group $dh_{ij}$ are set left-justified for matrix components of a second row in the $(2 \times n)$-dimensional text index value group matrix, and values of all matrix components exceeding p from a left end of the second row are set to 0.

Note that an $(m \times p)$ -dimensional first index value matrix $DW_{SVD}$ may be generated by performing dimensional compression processing which will be described later in a fourth embodiment on an $(m \times n)$-dimensional first index value matrix DW, a $(2 \times p)$-dimensional text index value group matrix may be generated by vertically (column direction) arranging a text index value group $dw_{ij}$ (j = 1 to p) on an ith row contained in this dimensionally compressed first index value matrix $DW_{SVD}$ and a text index value group $dh_{ij}$ (j = 1 to p) on the same ith row contained in the second index value matrix DH, and a prediction model for predicting severity of dementia may be generated based on this text index value group matrix.

As yet another example, as in Fig. 8(c), a text index value group $dw_{ij}$ on the ith row contained in the first index value matrix DW between texts/words is set to a $(1 \times n)$-dimensional first text index value group matrix, and a text index value group $dh_{ij}$ on the same ith row contained in the second index value matrix DH between texts/parts of speech is set to an $(n \times 1)$-dimensional second text index value group matrix (however, a value of a matrix component corresponding to a surplus of p and a shortage of n is set to 0), thereby calculating an inner product of the first text index value group matrix and the second text index value group matrix. Then, a prediction model for predicting severity of dementia may be generated based on the calculated value.

In this case, the $(m \times p)$-dimensional first index value matrix $DW_{SVD}$ may be generated by dimensionally compressing the first index value matrix DW between texts/words, and the inner product of the first text index value group matrix and the second text index value group matrix may be calculated by setting the text index value group $dw_{ij}$ on the ith row contained in this dimensionally compressed first index value matrix $DW_{SVD}$ to a $(1 \times p)$-dimensional first text index value group matrix and setting the text index value group $dh_{ij}$ on the same ith row contained in the second index value matrix DH between texts/parts of speech to a $(p \times 1)$-dimensional second text index value group matrix.

The dementia prediction unit 21C applies a relationship index value obtained by executing processes of the word extraction unit 11A, the part-of-speech extraction unit 11B, the text vector computation unit 121, the word vector computation unit 122, the part-of-speech vector computation unit 123, and the index value computation unit 13C on prediction data input by the prediction data input unit 20 to a prediction model generated by the prediction model generation unit 14C (prediction model stored in the prediction model storage unit 30C), thereby predicting severity of dementia for m' patients subjected to prediction.

As described in detail above, in the third embodiment, m texts representing content of a free conversation conducted by a patient whose severity of dementia is known are input as learning data, an inner product of a text vector computed from the input texts and a word vector computed from words contained in the texts is calculated to compute a relationship index value reflecting a relationship between the texts and the words, an inner product of a text vector computed from the input texts and a part-of-speech vector computed from parts of speech of morphemes contained in the texts is calculated to compute a relationship index value reflecting a relationship between the texts and the parts of speech, and a prediction model is generated using these relationship index values . In addition, when severity of dementia is predicted for a patient subjected to prediction, m' texts representing content of a free conversation conducted by the patient subjected to prediction are input as prediction data, and a relationship index value similarly computed from the input prediction data is applied to a prediction model, thereby predicting severity of dementia of the patient subjected to prediction.

Also in the third embodiment configured in this way, since the severity of dementia is predicted by analyzing the free conversation conducted by the patient, it is unnecessary to perform the mini-mental state examination (MMSE) . For this reason, even when the severity of dementia is repeatedly measured, it is possible to obtain a measurement result (prediction result) excluding the practice effect by the patient. In particular, in the third embodiment, since a relationship index value is computed in a state where a conversational characteristic peculiar to dementia is computed for a word and a part of speech used during a free conversation, and a prediction model is generated using the relationship index value, it is possible to more accurately predict the severity of dementia from the free conversation conducted by the patient.

(Fourth embodiment)

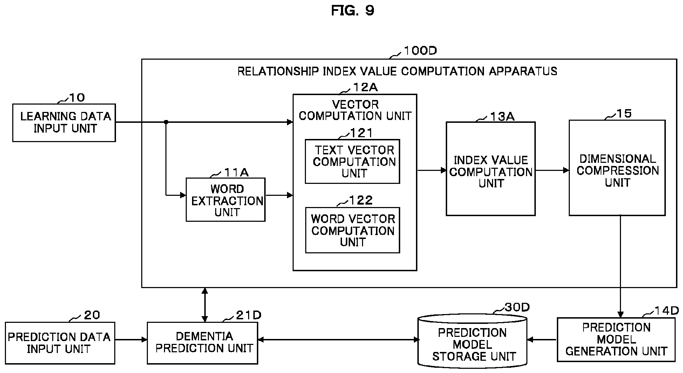

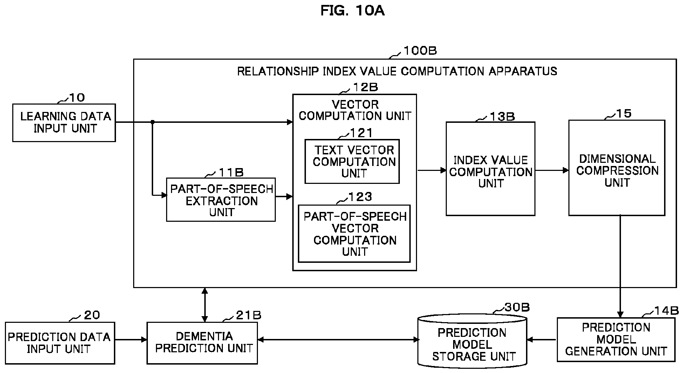

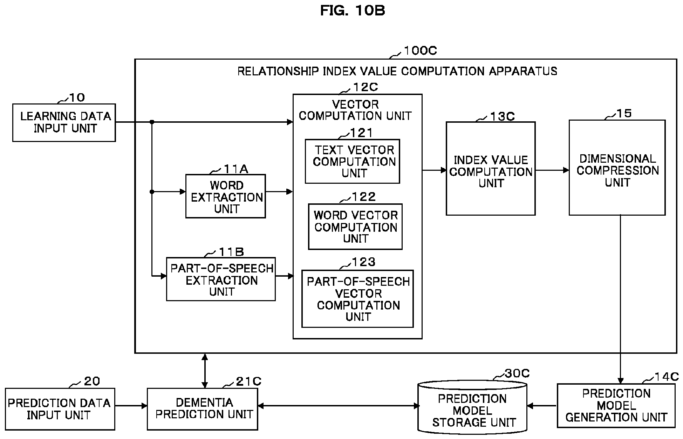

Next, a fourth embodiment according to the invention will be described with reference to the drawings. Fig. 9 is a block diagram illustrating a functional configuration example of a dementia prediction device according to the fourth embodiment. In Fig. 9, a component denoted by the same reference symbol as that illustrated in Fig. 1 has the same function, and thus duplicate description will be omitted here. Note that hereinafter, the fourth embodiment will be described as a modification to the first embodiment. However, as illustrated in each of Figs. 10A and 10B, the fourth embodiment can be similarly applied as a modification to the second embodiment or a modification to the third embodiment.

As illustrated in Fig. 9, the dementia prediction device according to the fourth embodiment includes a relationship index value computation unit 100D, a prediction model generation unit 14D, a dementia prediction unit 21D, and a prediction model storage unit 30D instead of the relationship index value computation unit 100A, the prediction model generation unit 14A, the dementia prediction unit 21A, and the prediction model storage unit 30A. The relationship index value computation unit 100D according to the fourth embodiment further includes a dimensional compression unit 15 in addition to the configuration illustrated in Fig. 1. Note that a prediction model generation device of the invention includes the learning data input unit 10, the relationship index value computation unit 100D, and the prediction model generation unit 14D.

The dimensional compression unit 15 performs predetermined dimensional compression processing using m × n relationship index values computed by the index value computation unit 13A, thereby computing m × k relationship index values (k is an arbitrary integer satisfying $1 \leq k < n$). In the dimensional compression processing, for example, known singular value decomposition (SVD) may be used as a method for decomposing a matrix.

That is, the dimensional compression unit 15 decomposes the index value matrix DW computed as in the above Equation (3) into three matrices U, S, and V. Here, the matrix U is an (m × k)-dimensional left singular matrix, in which each column is an eigenvector of $DW*DW^t$ ($DW^t$ denotes the transposed matrix of the index value matrix DW). The matrix S is a (k × k)-dimensional square matrix, in which a diagonal matrix component indicates a singular value of the index value matrix DW, and all other values are 0. The matrix V is a (k × n)-dimensional right singular matrix, in which each row is an eigenvector of $DW^t*DW$. Note that the dimension k after compression may be a fixed value determined in advance, or an arbitrary value may be specified.

The dimensional compression unit 15 compresses the dimension of the index value matrix DW by transforming the index value matrix DW by the transposed matrix $V^t$ of the right singular matrix V among the three matrices decomposed as described above. That is, by calculating an inner product of the (m × n)-dimensional index value matrix DW and the (n × k)-dimensional right singular transposed matrix $V^t$, the (m × n)-dimensional index value matrix DW is dimensionally compressed into the (m × k) -dimensional index value matrix $DW_{SVD}$ ($DW_{SVD} = DW*V^t$). Note that $DW_{SVD}$ denotes a matrix obtained by dimensionally compressing the index value matrix DW using the SVD, and a relationship of $DW \approx U*S*V = DW_{SVD}*V$ is established.

By compressing the dimension of the index value matrix DW using the SVD method in this way, the index value matrix DW can be low-rank approximated without impairing a characteristic represented by the index value matrix DW as much as possible. Here, an example of transforming the index value matrix DW by the transposed matrix $V^t$ of the right singular matrix V has been described. However, when the value of m is identical to the value of n, the index value matrix DW may be transformed by the left singular matrix U ($DW_{SVD} = DW*U$).

The prediction model generation unit 14D generates a prediction model for predicting severity of dementia based on a text index value group including k relationship index values $dw_{ij}$ (i = 1, 2, ..., k) for one text $d_i$ using m × k relationship index values dimensionally compressed by the dimensional compression unit 15. Then, the prediction model generation unit 14D causes the prediction model storage unit 30D to store the generated prediction model.

The dementia prediction unit 21D applies a relationship index value obtained by executing processes of the word extraction unit 11A, the text vector computation unit 121, the word vector computation unit 122, the index value computation unit 13A, and the dimensional compression unit 15 on prediction data input by the prediction data input unit 20 to a prediction model generated by the prediction model generation unit 14D (prediction model stored in the prediction model storage unit 30D), thereby predicting severity of dementia for m' patients subjected to prediction.

In the first embodiment, it is necessary to select a value of n by presuming a standard type of word spoken by one patient in a free conversation in the form of an interview of about 5 to 10 minutes. When the value of n is small, the word spoken by the one patient subjected to prediction and n types of words extracted from a text of learning data are less overlapped, and there is a possibility that there will be no overlap. In addition, information about a word not included in the n words (word not extracted by the word extraction unit 11) is not added to the index value matrix DW. For this reason, as the value of n decreases, accuracy of prediction decreases. Meanwhile, when a sufficiently large value of n is selected, a possibility that there will be no overlap decreases, and fewer words are not included in the n words. However, a size of the matrix increases, and the amount of calculation increases. In addition, a word having a low appearance frequency is included as a feature quantity, and overfitting is likely to occur.

On the other hand, according to the fourth embodiment, it is possible to extract a lot of (for example, all) words included in m texts as n words to generate an index value matrix DW, and it is possible to compute an index value matrix $DW_{SVD}$ that is dimensionally compressed in a state where a characteristic expressed by this index value matrix DW is reflected. According to this, a prediction model is generated by learning, and severity of dementia is predicted using the generated prediction, more accurately with a small calculation load.

Note that, here, an example using the SVD as an example of dimensional compression has been described. However, the invention is not limited thereto. For example, other dimensional compression methods such as principal component analysis (PCA) may be used.

In addition, in Fig. 9, a description has been given of an example of dimensionally compressing the index value matrix DW between texts/words generated in the first embodiment. However, similar operation can be performed in the case of dimensionally compressing the index value matrix DH between texts/parts of speech generated in the second embodiment as in Fig. 10A. On the other hand, an operation can be performed in the following mode in the case of dimensionally compressing the first index value matrix DW and the second index value matrix DH generated in the third embodiment as in Fig. 10B.

For example, it is possible to individually perform dimensional compression on each of the first index value matrix DW and the second index value matrix DH. That is, an (m × n)-dimensional first index value matrix DW is dimensionally compressed into an (m × k) -dimensional first index value matrix $DW_{SVD}$, and an (m × p) -dimensional second index value matrix DH is dimensionally compressed into an (m × k)-dimensional second index value matrix $DH_{SVD}$. As another example, as illustrated in Fig. 8 (a), the first index value matrix DW and the second index value matrix DH may be horizontally arranged to generate one m × (n + p) -dimensional index value matrix, and this generated index value matrix may be dimensionally compressed into an (m × k) -dimensional index value matrix.

(Fifth embodiment)

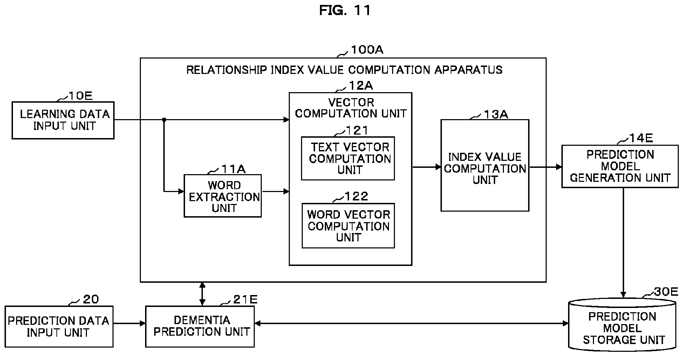

Next, a fifth embodiment according to the invention will be described with reference to the drawings . Fig. 11 is a block diagram illustrating a functional configuration example of a dementia prediction device according to the fifth embodiment. In Fig. 11, a component denoted by the same reference symbol as that illustrated in Fig. 1 has the same function, and thus duplicate description will be omitted here. Note that hereinafter, the fifth embodiment will be described as a modification to the first embodiment. However, the fifth embodiment can be similarly applied as a modification to any one of the second embodiment to the fourth embodiment.

As illustrated in Fig. 11, the dementia prediction device according to the fifth embodiment includes a learning data input unit 10E, a prediction model generation unit 14E, a dementia prediction unit 21E, and a prediction model storage unit 30E instead of the learning data input unit 10, the prediction model generation unit 14A, the dementia prediction unit 21A, and the prediction model storage unit 30A. Note that a prediction model generation device of the invention includes the learning data input unit 10E, the relationship index value computation unit 100A, and the prediction model generation unit 14E.

The learning data input unit 10E inputs, as learning data, m texts representing contents of free conversations conducted by m patients whose severity is known, respectively, for each of a plurality of evaluation items of dementia. The severity of the dementia for each of the plurality of evaluation items means a value of each score of each of five evaluation items of the MMSE, that is, each of insight, memory, attention (calculation), linguistic ability, and composition ability (graphical ability).

The prediction model generation unit 14E generates a prediction model for predicting severity of dementia for each evaluation item based on a text index value group including n relationship index values $dw_{ij}$ (j = 1, 2, ..., n) for one text $d_i$ using m × n relationship index values computed by the relationship index value computation unit 100A. Here, the severity of dementia to be predicted is a value of a score of each of five evaluation items of the MMSE.

That is, the prediction model generation unit 14E generates a prediction model in which a score as close as possible to ×1 point, ×2 points, ×3 points, ×4 points, and ×5 points is predicted for each evaluation item for a text index value group computed based on a free conversation of a patient whose score of each of insight, memory, attention, linguistic ability, and composition ability of the MMSE (for example, ×1 point, ×2 points, ×3 points, ×4 points, and ×5 points, respectively) is known. Then, the prediction model generation unit 14E causes the prediction model storage unit 30E to store the generated prediction model.

The prediction model generation unit 14E computes a feature quantity associated with severity of dementia for each evaluation item for each text index value group of each text $d_i$ (i = 1, 2, ..., m) using m × n relationship index values $dw_{11}$ to $dw_{mn}$ computed by the index value computation unit 13A for each evaluation item, and generates a prediction model for predicting severity of dementia for each evaluation item from one text index value group based on the computed feature quantity. Here, the prediction model generated by the prediction model generation unit 14E is a learning model in which a text index value group of a text $d_i$ is input and a score for each evaluation item of the MMSE is output as a solution.

Also in the fifth embodiment, the feature quantity computed when the prediction model generation unit 14E generates the prediction model may be computed by a predetermined algorithm. In other words, a method of computing the feature quantity performed by the prediction model generation unit 14E can be arbitrarily designed. For example, the prediction model generation unit 14E performs predetermined weighting calculation on each text index value group of each text $d_i$ for each evaluation item so that a value obtained by weighting calculation approaches a known value representing the severity of the dementia for each evaluation item (score for each evaluation item of the MMSE), and generates a prediction model for predicting the severity of dementia for each evaluation item (score for each evaluation item of the MMSE) from the text index value group of the text $d_i$ using a weighted value for the text index value group as the feature quantity for each evaluation item.

For example, the prediction model generation unit 14E generates a prediction model in which a score of a first evaluation item (insight) is predicted using one or more weight values among n weight values $\{a_{i1}, a_{i2}, ..., a_{in}\}$ for a text index value group of a text $d_i$ as a feature quantity, a score of a second evaluation item (memory) is predicted using another one or more weight values as a feature quantity, and similarly, scores of a third evaluation item to a fifth evaluation item (attention, linguistic ability, and composition ability) are predicted using yet another one or more weight values as a feature quantity.

The dementia prediction unit 21E applies a relationship index value obtained by executing processes of the word extraction unit 11A, the text vector computation unit 121, the word vector computation unit 122, and the index value computation unit 13A on prediction data input by the prediction data input unit 20 to a prediction model generated by the prediction model generation unit 14E (prediction model stored in the prediction model storage unit 30E), thereby predicting severity of dementia for each evaluation item for m' patients subjected to prediction.

According to the fifth embodiment configured as described above, it is possible to predict the score for each evaluation item of the MMSE without performing the mini-mental state examination (MMSE).

Note that even though a description has been given of an example of predicting the score for each of the five evaluation items of the MMSE here, the score may be predicted for each of more evaluation items obtained by further subdividing the five evaluation items.

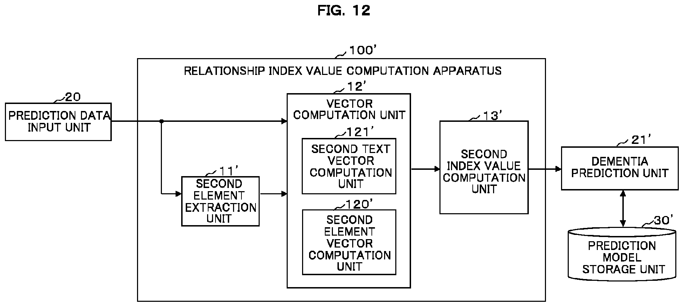

In the first to fifth embodiments, the dementia prediction device including a learner and a predictor has been illustrated. However, it is possible to separately configure a prediction model generation device having only the learner and a dementia prediction device having only the predictor. A configuration of the prediction model generation device including only the learner is as described in the first to fifth embodiments. On the other hand, for example, a configuration of the dementia prediction device including only the predictor is as illustrated in Fig. 12.

In Fig. 12, a second element extraction unit 11' has a similar function to that of any of the word extraction unit 11A, the part-of-speech extraction unit 11B, or a combination of the word extraction unit 11A and the part-of-speech extraction unit 11B. A second text vector computation unit 121' has a similar function to that of the text vector computation unit 121. A second element vector computation unit 120' has a similar function to that any of the word vector computation unit 122, the part-of-speech vector computation unit 123, or a combination of the word vector computation unit 122 and the part-of-speech vector computation unit 123. A second index value computation unit 13' has a similar function to that of any of the index value computation units 13A to 13E. A dementia prediction unit 21' has a similar function to that of any of the dementia prediction units 21A to 21E. A prediction model storage unit 30' stores a prediction model similar to that of any of the prediction model storage units 30A to 30E.

Further, in the first to fifth embodiments, a description has been given of an example of a case where the "severity of dementia" is the score for the MMSE, that is, an example of predicting the score for the MMSE. However, the invention is not limited thereto. For example, the severity of dementia may be set to categories classified by the number less than a maximum value of the score for the MMSE and greater than or equal to 2. For example, the severity of dementia may be classified into three categories such that there is no suspicion of dementia when the MMSE score is 30 to 27 points, mild dementia disorder is suspected when the MMSE score is 26 to 22 points, and dementia is suspected when the MMSE score is 21 points or less, and a category to which the patient corresponds may be predicted.

In this case, for example, in the first embodiment, the prediction model generation unit 14A generates a prediction model in which a text index value group computed based on text data corresponding to a free conversation of a patient whose MMSE score is known to be 30 to 27 points is classified into a first category of "there is no suspicion of dementia", a text index value group computed based on text data corresponding to a free conversation of a patient whose MMSE score is known to be 26 to 22 points is classified into a second category of "mild dementia disorder is suspected", and a text index value group computed based on text data corresponding to a free conversation of a patient whose MMSE score is known to be less than 21 points is classified into a third category of "dementia is suspected".

For example, the prediction model generation unit 14A computes each feature quantity for a text index value group of each text $d_i$, and optimizes categorization by the Markov chain Monte Carlo method according to a value of the computed feature quantity, thereby generating a prediction model for classifying each text $d_i$ into a plurality of categories. Here, the prediction model generated by the prediction model generation unit 14A is a learning model that inputs a text index value group and outputs any one of a plurality of categories to be predicted as a solution. Alternatively, the prediction model may be a learning model that outputs a probability of being classified into any category as a numerical value. The form of the learning model is arbitrary.

Further, in the first to fifth embodiments, a description has been given of an example of predicting the severity of dementia based on the MMSE score. However, the invention is not limited thereto. That is, it is possible to predict the severity of dementia based on a method of detecting the severity of dementia other than the MMSE score, for example, the revised Hasegawa's Dementia Scale-Revised (HDS-R), Alzheimer's Disease Assessment Scale-cognitive subscale (ADAS-cog), Clinical Dementia Rating (CDR), Clock Drawing Test (CDT), Neurobehavioral Cognitive Status Examination (COGNISTAT), Seven Minutes Screening, etc.

Further, in the first to fifth embodiments, a description has been given of an example in which a free conversation between a doctor and a patient in the form of an interview is converted into character data and used for learning and prediction related to the severity of dementia. However, the invention is not limited thereto. For example, a free conversation conducted by a patient in daily life may be converted into character data and used for learning and prediction related to the severity of dementia.

Reference Signs List

10, 10E Learning data input unit
11A Word extraction unit (element extraction unit)
11B Part-of-speech extraction unit (element extraction unit)
12A to 12E Vector computation unit
121 Text vector computation unit (element vector computation unit)
122 Word vector computation unit (element vector computation unit)
123 Part-of-speech vector computation unit (element vector computation unit)
13A to 13C Index value computation unit
14A to 14E Prediction model generation unit
15 Dimensional compression unit
20 Prediction data input unit
21A to 21E Dementia prediction unit
30A to 30E Prediction model storage unit
100A to 100E Relationship index value computation unit

**Claims**

1. A prediction model generation device comprising:

    a learning data input unit (10) that inputs a plurality of texts representing contents of free conversations conducted by a plurality of patients whose severity of dementia is known, respectively, as learning data;
    an element extraction unit (11A, 11B) that analyzes morphemes of the plurality of texts input by the learning data input unit (10) as the learning data, and extracts at least one of a plurality of words and a plurality of parts of speech as a plurality of decomposition elements from the plurality of texts, each in association with an appearance frequency;
    a text vector computation unit (121) that converts each of the plurality of texts into a q-dimensional vector, wherein q is an arbitrary integer of 2 or more, according to a predetermined rule, thereby computing a plurality of text vectors including q axis components;
    an element vector computation unit (122, 123) that converts each of the plurality of decomposition elements into a q-dimensional vector according to a predetermined rule, thereby computing a plurality of element vectors including q axis components;
    an index value computation unit (13A, 13B, 13C) that obtains each of inner products of the plurality of text vectors and the plurality of element vectors, thereby computing relationship index values reflecting a relationship between the plurality of texts and the plurality of decomposition elements; and
    a prediction model generation unit (14A, 14B, 14C, 14D, 14E) that generates a prediction model which is machine-learned so that a value indicating the severity of the dementia is output when a text index value group including a plurality of relationship index values for one text is input using the relationship index values computed by the index value computation unit (13A, 13B, 13C),
    wherein the value indicating the severity of the dementia is a value of a score of a mini-mental state examination or a value indicating a category classified by a number larger than 2 and less than a maximum value of the score of the mini-mental state examination.

2. The prediction model generation device according to claim 1,

    wherein the learning data input unit (10) inputs, as learning data, a plurality of texts representing contents of free conversations conducted by a plurality of patients whose severity is known, respectively, for each of a plurality of evaluation items of the dementia, and
    the prediction model generation unit (14A, 14B, 14C, 14D, 14E) generates a prediction model for obtaining output of the value indicating severity for each of the evaluation items of the dementia based on the text index value group.

3. A dementia prediction device including the prediction model generation device according to claim 1, and further comprising:

a prediction data input unit (20) that inputs a text representing content of a free conversation conducted by a patient subjected to prediction as prediction data; and

a dementia prediction unit (21A, 21B, 21C, 21D, 21E) that obtains output of a value indicating the severity of the dementia for the patient subjected to prediction by inputting a text index value group obtained by executing processes of the element extraction unit (11A, 11B), the text vector computation unit (121), the element vector computation unit (122, 123), and the index value computation unit (13A, 13B, 13C) on the prediction data input by the prediction data input unit (20) to the prediction model generated by the prediction model generation unit (14A, 14B, 14C, 14D, 14E).

4. The dementia prediction device according to claim 3,

wherein the learning data input unit (10) inputs, as the learning data, m texts representing contents of free conversations conducted by m patients, wherein m is an arbitrary integer of 2 or more, whose severity of dementia is known, respectively,

the element extraction unit (11A, 11B) is a word extraction unit (11A) that analyzes the m texts input as the learning data by the learning data input unit (10) and extracts n words, wherein n is an arbitrary integer of 2 or more, from the m texts, in association with an appearance frequency of the words,

the text vector computation unit (121) converts each of the m texts into a q-dimensional vector according to a predetermined rule, thereby computing m text vectors including q axis components,

the element vector computation unit (122, 123) is a word vector computation unit (122) that converts each of the n words into a q-dimensional vector according to a predetermined rule, thereby computing n word vectors including q axis components,

the index value computation unit (13A) obtains a product of a text matrix having the respective q axis components of the m text vectors as respective elements and a word matrix having the respective q axis components of the n word vectors as respective elements, thereby computing an index value matrix having $m \times n$ relationship index values reflecting a relationship between the m texts and the n words as respective elements,

the prediction model generation unit (14A, 14D, 14E) generates a prediction model which is machine-learned so that the value indicating the severity of the dementia is output when the text index value group including n relationship index values included in the identical row of the index value matrix is input using the index value matrix computed by the index value computation unit (13A),

the prediction data input unit (20) inputs, as prediction data, m' texts representing contents of free conversations conducted by m' patients, wherein m' is an arbitrary integer of 1 or more, subjected to prediction, respectively, and

the dementia prediction unit (21A, 21D, 21E) obtains output of the value indicating the severity of the dementia for the m' patients subjected to prediction by inputting m' text index value groups obtained by executing processes of the word extraction unit (11A), the text vector computation unit (121), the word vector computation unit (122), and the index value computation unit (13A) on the prediction data input by the prediction data input unit (20) to the prediction model generated by the prediction model generation unit (14A, 14D, 14E).

5. The dementia prediction device according to claim 3,

wherein the learning data input unit (10) inputs, as the learning data, m texts representing contents of free conversations conducted by m patients, wherein m is an arbitrary integer of 2 or more, whose severity of dementia is known, respectively,

the element extraction unit (11A, 11B) is a part-of-speech extraction unit (11B) that analyzes the m texts input as the learning data by the learning data input unit (10) and extracts p parts of speech, wherein p is an arbitrary integer of 2 or more, from the m texts, in association with an appearance frequency of the parts of speech,

the text vector computation unit (121) converts each of the m texts into a q-dimensional vector according to a predetermined rule, thereby computing m text vectors including q axis components,

the element vector computation unit (122, 123) is a part-of-speech vector computation unit (123) that converts the p parts of speech into a q-dimensional vector according to a predetermined rule, thereby computing p part-of-speech vectors including q axis components,

the index value computation unit (13B) obtains a product of a text matrix having the respective q axis components of the m text vectors as respective elements and a part-of-speech matrix having the respective q axis components of the p part-of-speech vectors as respective elements, thereby computing an index value matrix having $m \times p$ relationship index values reflecting a relationship between the m texts and the p parts of speech as respective elements,

the prediction model generation unit (14B, 14D, 14E) generates a prediction model which is machine-learned so that the value indicating the severity of the dementia is output when the text index value group including p relationship index values included in the identical row of the index value matrix is input using the index value matrix computed by the index value computation unit (13B),

the prediction data input unit (20) inputs, as prediction data, m' texts representing contents of free conversations conducted by m' patients, wherein m' is an arbitrary integer of 1 or more, subjected to prediction, respectively, and the dementia prediction unit (21B, 21D, 21E) obtains output of the value indicating the severity of the dementia for the m' patients subjected to prediction by inputting m' text index value groups obtained by executing processes of the part-of-speech extraction unit (11B), the text vector computation unit (121), the part-of-speech vector computation unit (123), and the index value computation unit (13B) on the prediction data input by the prediction data input unit (20) to the prediction model generated by the prediction model generation unit (14B, 14D, 14E).

6. The dementia prediction device according to claim 3,

wherein the learning data input unit (10) inputs, as the learning data, m texts representing contents of free conversations conducted by m patients, wherein m is an arbitrary integer of 2 or more, whose severity of dementia is known, respectively,

the element extraction unit (11A, 11B) includes a word extraction unit (11A) that analyzes the m texts input as the learning data by the learning data input unit (10) and extracts n words, wherein n is an arbitrary integer of 2 or more, from the m texts, in association with an appearance frequency of the words, and a part-of-speech extraction unit (11B) that analyzes the m texts input as the learning data by the learning data input unit (10) and extracts p parts of speech, wherein p is an arbitrary integer of 2 or more, from the m texts, in association with an appearance frequency of the parts of speech,

the text vector computation unit (121) converts each of the m texts into a q-dimensional vector according to a predetermined rule, thereby computing m text vectors including q axis components,

the element vector computation unit (122, 123) includes a word vector computation unit (122) that converts each of the n words into a q-dimensional vector according to a predetermined rule, thereby computing n word vectors including q axis components, and a part-of-speech vector computation unit (123) that converts each of the p parts of speech into a q-dimensional vector according to a predetermined rule, thereby computing p part-of-speech vectors including q axis components,

the index value computation unit (13C) obtains a product of a text matrix having the respective q axis components of the m text vectors as respective elements and a word matrix having the respective q axis components of the n word vectors as respective elements, thereby computing a first index value matrix having $m \times n$ relationship index values reflecting a relationship between the m texts and the n words as respective elements, and obtains a product of the text matrix having the respective q axis components of the m text vectors as respective elements and a part-of-speech matrix having the respective q axis components of the p part-of-speech vectors as respective elements, thereby computing a second index value matrix having $m \times p$ relationship index values reflecting a relationship between the m texts and the p parts of speech as respective elements,

the prediction model generation unit (14C, 14D, 14E) generates a prediction model which is machine-learned so that the value indicating the severity of the dementia is output when a first text index value group including n relationship index values included in the identical row of the first index value matrix and a second text index value group including p relationship index values included in the identical row of the second index value matrix are input using the first index value matrix and the second index value matrix computed by the index value computation unit (13C),

the prediction data input unit (20) inputs, as prediction data, m' texts representing contents of free conversations conducted by m' patients, wherein m' is an arbitrary integer of 1 or more, subjected to prediction, respectively, and the dementia prediction unit (21C, 21D, 21E) obtains output of the value indicating the severity of the dementia for the m' patients subjected to prediction by inputting m' first text index value groups and m' second text index value groups obtained by executing processes of the word extraction unit (11A), the part-of-speech extraction unit (11B), the text vector computation unit (121), the word vector computation unit (122), the part-of-speech vector computation unit (123), and the index value computation unit (13C) on the prediction data input by the prediction data input unit (20) to the prediction model generated by the prediction model generation unit (14C, 14D, 14E).

7. The dementia prediction device according to any one of claims 4 to 6, further comprising

a dimensional compression unit (15) that performs predetermined dimensional compression processing on the index value matrix computed by the index value computation unit (13A, 13B, 13C), thereby computing a dimensionally compressed index value matrix,

wherein the prediction model generation unit (14A, 14B, 14C, 14D, 14E) generates a prediction model which is machine-learned so that the value indicating the severity of the dementia is output when the text index value group including the plurality of relationship index values included in the identical row of the index value matrix is input using an index value matrix dimensionally compressed by the dimensional compression unit (15), and the dementia prediction unit (21A, 21B, 21C, 21D, 21E) inputs the text index value group extracted from the dimensionally compressed index value matrix obtained by further executing the processing of the dimensional compression unit (15) on the index value matrix computed by the index value computation unit (13A, 13B, 13C) to the prediction model generated by the prediction model generation unit (14A, 14B, 14C, 14D, 14E), thereby obtains output of the value indicating the severity of the dementia for the patient subjected to prediction.

8. The dementia prediction device according to any one of claims 3 to 7, wherein the prediction model generation unit (14A, 14B, 14C, 14D, 14E) computes a feature quantity associated with the severity of the dementia for the text index value group, and generates the prediction model for obtaining output of the value indicating the severity of the dementia from the text index value group based on the computed feature quantity.

9. The dementia prediction device according to claim 8, wherein the prediction model generation unit (14A, 14B, 14C, 14D, 14E) performs predetermined weighting calculation on the text index value group so that a value obtained by weighting calculation approaches a known value representing the severity of the dementia, and generates the prediction model for obtaining output of the value indicating the severity of the dementia from the text index value group using a weighted value for the text index value group as the feature quantity.

10. The dementia prediction device according to any one of claims 3 to 7,

wherein the learning data input unit (10) inputs, as learning data, a plurality of texts representing contents of free conversations conducted by a plurality of patients whose severity is known, respectively, for each of a plurality of evaluation items of the dementia,
the prediction model generation unit (14A, 14B, 14C, 14D, 14E) generates a prediction model for obtaining output of the value indicating severity for each of the evaluation items of the dementia based on the text index value group, and
the dementia prediction unit (21A, 21B, 21C, 21D, 21E) obtains output of the value indicating the severity for each of the evaluation items of the dementia for the patient subjected to prediction.

11. The dementia prediction device according to claim 10, wherein the prediction model generation unit (14A, 14B, 14C, 14D, 14E) computes a feature quantity associated with severity for each of the evaluation items of the dementia for each of the evaluation items for the text index value group, and generates the prediction model for obtaining output of the value indicating severity for each of the evaluation items of the dementia from the text index value group based on the computed feature quantity.

12. The dementia prediction device according to claim 11, **characterized in that** the prediction model generation unit (14A, 14B, 14C, 14D, 14E) performs predetermined weighting calculation on the text index value group so that a value obtained by weighting calculation for each of the evaluation items approaches a known value representing the severity for each of the evaluation items of the dementia, and generates the prediction model for obtaining output of the value indicating the severity for each of the evaluation items of the dementia from the text index value group using a weighted value for the text index value group as the feature quantity for each of the evaluation items.

13. A dementia prediction device comprising:

a prediction data input unit (20) that inputs one or more texts representing content of a free conversation conducted by a patient subjected to prediction as prediction data;
a second element extraction unit (11') that analyzes morphemes of the one or more texts input by the prediction data input unit (20) as the prediction data, and extracts at least one of a plurality of words and a plurality of parts of speech as a plurality of decomposition elements from the one or more texts, each in association with an appearance frequency;
a second text vector computation unit (121') that converts the one or more texts into a q-dimensional vector, wherein q is an arbitrary integer of 2 or more, according to a predetermined rule, thereby computing one or more text vectors including q axis components;
a second element vector computation unit (120') that converts each of the plurality of decomposition elements into a q-dimensional vector according to a predetermined rule, thereby computing a plurality of element vectors including q axis components;

a second index value computation unit (13') that obtains each of inner products of the one or more text vectors and the plurality of element vectors, thereby computing relationship index values reflecting a relationship between the one or more texts and the plurality of decomposition elements; and

a dementia prediction unit (21') that applies a relationship index value computed by the second index value computation unit (13') to a prediction model generated by the prediction model generation device according to claim 1, thereby obtaining output of the value indicating the severity of the dementia for the patient subjected to prediction,

wherein the value indicating the severity of the dementia is a value of a score of a mini-mental state examination or a value indicating a category classified by a number larger than 2 and less than a maximum value of the score of the mini-mental state examination.

14. A dementia prediction program that causes a computer to function as:

learning data input means that inputs a plurality of texts representing contents of free conversations conducted by a plurality of patients whose severity of dementia is known, respectively, as learning data;

element extraction means that analyzes morphemes of the plurality of texts input by the learning data input means as the learning data, and extracts at least one of a plurality of words and a plurality of parts of speech as a plurality of decomposition elements from the plurality of texts, each in association with an appearance frequency;

text vector computation means that converts each of the plurality of texts into a q-dimensional vector, wherein q is an arbitrary integer of 2 or more, according to a predetermined rule, thereby computing a plurality of text vectors including q axis components;

element vector computation means that converts each of the plurality of decomposition elements into a q-dimensional vector according to a predetermined rule, thereby computing a plurality of element vectors including q axis components;

index value computation means that obtains each of inner products of the plurality of text vectors and the plurality of element vectors, thereby computing relationship index values reflecting a relationship between the plurality of texts and the plurality of decomposition elements; and

prediction model generation means that generates a prediction model machine-learned so that a value indicating the severity of the dementia is output when a text index value group including a plurality of relationship index values for one text is input using the relationship index values computed by the index value computation means, wherein the value indicating the severity of the dementia is a value of a score of a mini-mental state examination or a value indicating a category classified by a number larger than 2 and less than a maximum value of the score of the mini-mental state examination.

15. The dementia prediction program according to claim 14, further causing the computer to function as:

prediction data input means that inputs a text representing content of a free conversation conducted by a patient subjected to prediction as prediction data; and

dementia prediction means that obtains output of a value indicating the severity of the dementia for the patient subjected to prediction by inputting a text index value group obtained by executing processes of the element extraction means, the text vector computation means, the element vector computation means, and the index value computation means on the prediction data input by the prediction data input means to the prediction model generated by the prediction model generation means.

16. A dementia prediction program that causes a computer to function as:

prediction data input means that inputs one or more texts representing content of a free conversation conducted by a patient subjected to prediction as prediction data;

second element extraction means that analyzes morphemes of the one or more texts input by the prediction data input means as the prediction data, and extracts at least one of a plurality of words and a plurality of parts of speech as a plurality of decomposition elements from the one or more texts, each in association with an appearance frequency;

second text vector computation means that converts the one or more texts into a q-dimensional vector, wherein q is an arbitrary integer of 2 or more, according to a predetermined rule, thereby computing one or more text vectors including q axis components;

second element vector computation means that converts each of the plurality of decomposition elements into a q-dimensional vector according to a predetermined rule, thereby computing a plurality of element vectors including q axis components;
second index value computation means that obtains each of inner products of the one or more text vectors and the plurality of element vectors, thereby computing relationship index values reflecting a relationship between the one or more texts and the plurality of decomposition elements; and
dementia prediction means that applies a relationship index value computed by the second index value computation means to a prediction model generated by the prediction model generation means according to claim 14, thereby obtaining output of the value indicating the severity of the dementia for the patient subjected to prediction.

**Patentansprüche**

1. Vorhersagemodell-Erzeugungsvorrichtung, die aufweist:

eine Lerndaten-Eingabeeinheit (10), die mehrere Texte, die Inhalte von freien Konversationen repräsentieren, die von mehreren Patienten geführt werden, deren Schweregrad der Demenz jeweils bekannt ist, als Lerndaten eingibt,
eine Elementextraktionseinheit (11A, 11B), die Morpheme der mehreren Texte, die von der Lerndaten-Eingabeeinheit (10) als die Lerndaten eingegeben werden, analysiert und mehrere Wörter und/oder mehrere Teile der Sprache als mehrere Zerlegungselemente aus den mehreren Texten extrahiert, jeweils in Verbindung mit einer Erscheinungshäufigkeit;
eine Textvektor-Berechnungseinheit (121), die jeden der mehreren Texte in einen q-dimensionalen Vektor, wobei q eine beliebige ganze Zahl von 2 oder mehr ist, gemäß einer vorbestimmten Regel umwandelt, wodurch mehrere Textvektoren berechnet werden, die q Achsenkomponenten enthalten,
eine Elementvektor-Berechnungseinheit (122, 123), die jedes der mehreren Zerlegungselemente gemäß einer vorbestimmten Regel in einen q-dimensionalen Vektor umwandelt, wodurch mehrere Elementvektoren mit q Achsenkomponenten berechnet werden,
eine Indexwert-Berechnungseinheit (13A, 13B, 13C), die jeweils innere Produkte der mehreren Textvektoren und der mehreren Elementvektoren erhält, wodurch Beziehungsindexwerte berechnet werden, die eine Beziehung zwischen den mehreren Texten und den mehreren Zerlegungselementen wiedergeben, und
eine Vorhersagemodell-Erzeugungseinheit (14A, 14B, 14C, 14D, 14E), die ein Vorhersagemodell erzeugt, das maschinell erlernt ist, so dass ein Wert, der den Schweregrad der Demenz anzeigt, ausgegeben wird, wenn eine Textindexwertgruppe eingegeben wird, die mehrere Beziehungsindexwerte für einen Text enthält, unter Verwendung der Beziehungsindexwerte, die durch die Indexwert-Berechnungseinheit (13A, 13B, 13C) berechnet werden,
wobei der Wert, der den Schweregrad der Demenz anzeigt, ein Wert einer Auswertung einer Mini-Geisteszustandsprüfung oder ein Wert ist, der eine Kategorie anzeigt, die durch eine Zahl größer als 2 und kleiner als ein Maximalwert der Auswertung der Mini-Geisteszustandsprüfung klassifiziert ist.

2. Vorhersagemodell-Erzeugungsvorrichtung nach Anspruch 1, wobei die Lerndaten-Eingabeeinheit (10) als Lerndaten mehrere Texte eingibt, die Inhalte von freien Konversationen repräsentieren, die von mehreren Patienten geführt werden, deren Schweregrad der Demenz jeweils bekannt ist, für jeweils mehrere Bewertungselemente der Demenz, und
die Vorhersagemodell-Erzeugungseinheit (14A, 14B, 14C, 14D, 14E) ein Vorhersagemodell erzeugt, um eine Ausgabe des Wertes zu erhalten, der den Schweregrad für jeden der Bewertungspunkte der Demenz basierend auf der Textindexwertgruppe anzeigt.

3. Demenzvorhersagevorrichtung, die die Vorhersagemodell-Erzeugungsvorrichtung nach Anspruch 1 enthält, und die ferner aufweist:

eine Vorhersagedaten-Eingabeeinheit (20), die einen Text, der den Inhalt einer freien Konversation repräsentiert, die von einem der Vorhersage unterzogenen Patienten geführt wird, als Vorhersagedaten eingibt, und
eine Demenzvorhersageeinheit (21A, 21B, 21C, 21D, 21E), die die Ausgabe eines Wertes erhält, der den Schweregrad der Demenz für den der Vorhersage unterzogenen Patienten anzeigt, indem sie eine Textindexwertgruppe eingibt, die durch Ausführen von Prozessen der Elementextraktionseinheit (11A, 11B), der Textvektorberechnungseinheit (121), der Elementvektor-Berechnungseinheit (122, 123) und der Indexwert-Berechnungseinheit (13A, 13B, 13C) an den Vorhersagedaten erhalten wird, die durch die Vorhersagedaten-Eingabeeinheit (20) in das durch die Vorhersagemodell-Erzeugungseinheit (14A, 14B, 14C, 14D, 14E) erzeugte Vorhersagemodell eingegeben werden.

4. Demenzvorhersagevorrichtung nach Anspruch 3, wobei die Lerndaten-Eingabeeinheit (10) als die Lerndaten m Texte eingibt, die Inhalte von freien Konversationen repräsentieren, die von m Patienten geführt wurden, wobei m eine beliebige ganze Zahl von 2 oder mehr ist, deren Schweregrad der Demenz jeweils bekannt ist,

die Elementextraktionseinheit (11A, 11B) eine Wortextraktionseinheit (11A) ist, die die m Texte, die als die Lerndaten durch die Lerndaten-Eingabeeinheit (10) eingegeben werden, analysiert und n Wörter, wobei n eine beliebige ganze Zahl von 2 oder mehr ist, aus den m Texten in Verbindung mit einer Erscheinungshäufigkeit der Wörter extrahiert, die Textvektor-Berechnungseinheit (121) jeden der m Texte gemäß einer vorbestimmten Regel in einen q-dimensionalen Vektor umwandelt, wodurch m Textvektoren mit q Achsenkomponenten berechnet werden,

die Elementvektor-Berechnungseinheit (122, 123) eine Wortvektor-Berechnungseinheit (122) ist, die jedes der n Wörter gemäß einer vorbestimmten Regel in einen q-dimensionalen Vektor umwandelt, wodurch n Wortvektoren mit q Achsenkomponenten berechnet werden,

die Indexwert-Berechnungseinheit (13A) ein Produkt aus einer Textmatrix mit den jeweiligen q Achsenkomponenten der m Textvektoren als jeweilige Elemente und einer Wortmatrix mit den jeweiligen q Achsenkomponenten der n Wortvektoren als jeweilige Elemente erhält, wodurch eine Indexwertmatrix mit $m \times n$ Beziehungsindexwerten berechnet wird, die eine Beziehung zwischen den m Texten und den n Wörtern als jeweilige Elemente wiedergeben,

die Vorhersagemodell-Erzeugungseinheit (14A, 14D, 14E) ein Vorhersagemodell erzeugt, das maschinell erlernt ist, so dass der Wert, der den Schweregrad der Demenz anzeigt, ausgegeben wird, wenn die Textindexwertgruppe, die n Beziehungsindexwerte enthält, die in der identischen Reihe der Indexwertmatrix enthalten sind, unter Verwendung der Indexwertmatrix eingegeben wird, die von der Indexwert-Berechnungseinheit (13A) berechnet wurde,

die Vorhersagedaten-Eingabeeinheit (20) als Vorhersagedaten m' Texte eingibt, die Inhalte von freien Konversationen repräsentieren, die von m' Patienten geführt werden, wobei m' eine beliebige ganze Zahl von 1 oder mehr ist, die jeweils einer Vorhersage unterzogen werden, und

die Demenzvorhersageeinheit (21A, 21D, 21E) die Ausgabe des Wertes erhält, der den Schweregrad der Demenz für die m' der Vorhersage unterzogenen Patienten anzeigt, indem m' Textindexwertgruppen eingegeben werden, die durch Ausführen von Prozessen der Wortextraktionseinheit (11A), der Textvektorberechnungseinheit (121), der Wortvektorberechnungseinheit (122) und der Indexwert-Berechnungseinheit (13A) an den Vorhersagedaten erhalten werden, die durch die Vorhersagedaten-Eingabeeinheit (20) in das durch die Vorhersagemodell-Erzeugungseinheit (14A, 14D, 14E) erzeugte Vorhersagemodell eingegeben werden.

5. Demenzvorhersagevorrichtung nach Anspruch 3, wobei die Lerndaten-Eingabeeinheit (10) als die Lerndaten m Texte eingibt, die Inhalte von freien Konversationen repräsentieren, die von m Patienten geführt werden, wobei m eine beliebige ganze Zahl von 2 oder mehr ist, deren Schweregrad der Demenz jeweils bekannt ist,

die Elementextraktionseinheit (11A, 11B) eine Sprachteil-Extraktionseinheit (11B) ist, die die m Texte, die als die Lerndaten durch die Lerndaten-Eingabeeinheit (10) eingegeben werden, analysiert und p Sprachteile, wobei p eine beliebige ganze Zahl von 2 oder mehr ist, aus den m Texten in Verbindung mit einer Erscheinungshäufigkeit der Sprachteile extrahiert,

die Textvektor-Berechnungseinheit (121) jeden der m Texte gemäß einer vorbestimmten Regel in einen q-dimensionalen Vektor umwandelt, wodurch m Textvektoren mit q Achsenkomponenten berechnet werden,

die Elementvektor-Berechnungseinheit (122, 123) eine Sprachteilvektor-Berechnungseinheit (123) ist, die die p Sprachteile gemäß einer vorbestimmten Regel in einen q-dimensionalen Vektor umwandelt, wodurch p Sprachteilvektoren mit q Achsenkomponenten berechnet werden,

die Indexwert-Berechnungseinheit (13B) ein Produkt aus einer Textmatrix mit den jeweiligen q Achsenkomponenten der m Textvektoren als jeweilige Elemente und einer Sprachteilmatrix mit den jeweiligen q Achsenkomponenten der p Sprachteilvektoren als jeweilige Elemente erhält, wodurch eine Indexwertmatrix mit $m \times p$ Beziehungsindexwerten berechnet wird, die eine Beziehung zwischen den m Texten und den p Sprachteilen als jeweilige Elemente wiedergeben,

die Vorhersagemodell-Erzeugungseinheit (14B, 14D, 14E) ein Vorhersagemodell erzeugt, das maschinell erlernt ist, so dass der Wert, der den Schweregrad der Demenz anzeigt, ausgegeben wird, wenn die Textindexwertgruppe, die p Beziehungsindexwerte enthält, die in der identischen Zeile der Indexwertmatrix enthalten sind, unter Verwendung der Indexwertmatrix eingegeben wird, die von der Indexwert-Berechnungseinheit (13B) berechnet wird,

die Vorhersagedaten-Eingabeeinheit (20) als Vorhersagedaten m' Texte eingibt, die Inhalte von freien Konversationen repräsentieren, die von m' Patienten geführt werden, wobei m' eine beliebige ganze Zahl von 1 oder mehr ist, die jeweils einer Vorhersage unterzogen werden, und

die Demenzvorhersageeinheit (21B, 21D, 21E) eine Ausgabe des Wertes erhält, der den Schweregrad der Demenz für die m' der Vorhersage unterzogenen Patienten anzeigt, indem m' Textindexwertgruppen eingegeben werden, die durch Ausführen von Prozessen der Sprachteil-Extraktionseinheit (11B), der Textvektor-Berechnungseinheit (121), der Sprachteil-Vektor-Berechnungseinheit (123) und der Indexwert-Berechnungseinheit (13B) an den Vorhersagedaten erhalten werden, die durch die Vorhersagedaten-Eingabeeinheit (20) in das durch die Vorhersagemodell-Erzeugungseinheit (14B, 14D, 14E) erzeugte Vorhersagemodell eingegeben werden.

6. Demenzvorhersagevorrichtung nach Anspruch 3, wobei die Lerndaten-Eingabeeinheit (10) als die Lerndaten m Texte eingibt, die Inhalte von freien Konversationen repräsentieren, die von m Patienten geführt wurden, wobei m eine beliebige ganze Zahl von 2 oder mehr ist, deren Schweregrad der Demenz jeweils bekannt ist,

die Elementextraktionseinheit (11A, 11B) eine Wortextraktionseinheit (11A) enthält, die die m Texte, die als die Lerndaten durch die Lerndaten-Eingabeeinheit (10) eingegeben werden, analysiert und n Wörter, wobei n eine beliebige ganze Zahl von 2 oder mehr ist, aus den m Texten in Verbindung mit einer Erscheinungshäufigkeit der Wörter extrahiert, und eine Sprachteil-Extraktionseinheit (11B), die die m Texte analysiert, die als die Lerndaten durch die Lerndaten-Eingabeeinheit (10) eingegeben werden, und p Sprachteile aus den m Texten, wobei p eine beliebige ganze Zahl von 2 oder mehr ist, in Verbindung mit einer Erscheinungshäufigkeit der Sprachteile extrahiert,

die Textvektor-Berechnungseinheit (121) jeden der m Texte gemäß einer vorbestimmten Regel in einen q-dimensionalen Vektor umwandelt, wodurch m Textvektoren mit q Achsenkomponenten berechnet werden,

die Elementvektor-Berechnungseinheit (122, 123) eine Wortvektor-Berechnungseinheit (122) enthält, die jedes der n Wörter in einen q-dimensionalen Vektor gemäß einer vorbestimmten Regel umwandelt, wodurch n Wortvektoren mit q Achsenkomponenten berechnet werden, und eine Sprachteilvektor-Berechnungseinheit (123), die jeden der p Sprachteile in einen q-dimensionalen Vektor gemäß einer vorbestimmten Regel umwandelt, wodurch p Sprachteilvektoren mit q Achsenkomponenten berechnet werden, die Indexwert-Berechnungseinheit (13C) ein Produkt einer Textmatrix mit den jeweiligen q Achsenkomponenten der m Textvektoren als jeweilige Elemente und einer Wortmatrix mit den jeweiligen q Achsenkomponenten der n Wortvektoren als jeweilige Elemente erhält, wodurch eine erste Indexwertmatrix mit m × n Beziehungsindexwerten berechnet wird, die eine Beziehung zwischen den m Texten und den n Wörtern als jeweilige Elemente wiedergeben und ein Produkt der Textmatrix mit den jeweiligen q Achsenkomponenten der m Textvektoren als jeweilige Elemente und einer Sprachteilmatrix mit den jeweiligen q Achsenkomponenten der p Sprachteilvektoren als jeweilige Elemente erhält, wodurch eine zweite Indexwertmatrix mit m × p Beziehungsindexwerten, die eine Beziehung zwischen den m Texten und den p Sprachteilen als jeweilige Elemente wiedergeben, berechnet wird,

die Vorhersagemodell-Erzeugungseinheit (14C, 14D, 14E) ein Vorhersagemodell erzeugt, das maschinell erlernt ist, so dass der Wert, der den Schweregrad der Demenz anzeigt, ausgegeben wird, wenn eine erste Textindexwertgruppe, die n Beziehungsindexwerte enthält, die in der identischen Reihe der ersten Indexwertmatrix enthalten sind, und eine zweite Textindexwertgruppe, die p Beziehungsindexwerte enthält, die in der identischen Zeile der zweiten Indexwertmatrix enthalten sind, unter Verwendung der ersten Indexwertmatrix und der zweiten Indexwertmatrix, die von der Indexwert-Berechnungseinheit (13C) berechnet werden, eingegeben werden,

die Vorhersagedaten-Eingabeeinheit (20) als Vorhersagedaten m' Texte eingibt, die Inhalte von freien Konversationen repräsentieren, die von m' Patienten geführt werden, wobei m' eine beliebige ganze Zahl von 1 oder mehr ist, die jeweils einer Vorhersage unterzogen werden, und

die Demenzvorhersageeinheit (21C, 21D, 21E) die Ausgabe des Wertes erhält, der den Schweregrad der Demenz für die m' der Vorhersage unterzogenen Patienten anzeigt, indem m' erste Textindexwertgruppen und m' zweite Textindexwertgruppen eingegeben werden, die durch Ausführen von Prozessen der Wortextraktionseinheit (11A), der Sprachteil-Extraktionseinheit (11B), der Textvektor-Berechnungseinheit (121), der Wortvektor-Berechnungseinheit (122), der Sprachteil-Vektor-Berechnungseinheit (123) und der Indexwert-Berechnungseinheit (13C) an den Vorhersagedaten erhalten werden, die von der Vorhersagedaten-Eingabeeinheit (20) in das von der Vorhersagemodell-Erzeugungseinheit (14C, 14D, 14E) erzeugte Vorhersagemodell eingegeben werden.

7. Demenzvorhersagevorrichtung nach einem der Ansprüche 4 bis 6, die ferner aufweist:

eine Dimensionskompressionseinheit (15), die eine vorbestimmte Dimensionskompressionsverarbeitung an der von der Indexwert-Berechnungseinheit (13A, 13B, 13C) berechneten Indexwertmatrix durchführt, wodurch eine dimensionsmäßig komprimierte Indexwertmatrix berechnet wird,

wobei die Vorhersagemodell-Erzeugungseinheit (14A, 14B, 14C, 14D, 14E) ein Vorhersagemodell erzeugt, das maschinell erlernt ist, so dass der Wert, der den Schweregrad der Demenz anzeigt, ausgegeben wird, wenn die Textindexwertgruppe, die die mehreren Beziehungsindexwerte enthält, die in der identischen Zeile der Indexwertmatrix enthalten sind, unter Verwendung einer Indexwertmatrix, die durch die Dimensionskompressionseinheit (15) dimensionsmäßig komprimiert ist, eingegeben wird, und

die Demenzvorhersageeinheit (21A, 21B, 21C, 21D, 21E) die Textindexwertgruppe, die aus der dimensionsmäßig komprimierten Indexwertmatrix extrahiert wird, die durch weiteres Ausführen der Verarbeitung der Dimensionskompressionseinheit (15) an der durch die Indexwert-Berechnungseinheit (13A, 13B, 13C) berechneten Indexwertmatrix, in das von der Vorhersagemodell-Erzeugungseinheit (14A, 14B, 14C, 14D, 14E) erzeugte Vorhersagemodell eingibt, wodurch die Ausgabe des Wertes erhalten wird, der den Schweregrad der Demenz für den der Vorhersage unterzogenen Patienten anzeigt.

8. Demenzvorhersagevorrichtung nach einem der Ansprüche 3 bis 7, wobei die Vorhersagemodell-Erzeugungseinheit (14A, 14B, 14C, 14D, 14E) eine Merkmalsgröße, die mit dem Schweregrad der Demenz verbunden ist, für die Textindexwertgruppe berechnet und das Vorhersagemodell zum Erhalten der Ausgabe des Wertes, der den Schweregrad der Demenz anzeigt, aus der Textindexwertgruppe basierend auf der berechneten Merkmalsgröße erzeugt.

9. Demenzvorhersagevorrichtung nach Anspruch 8, wobei die Vorhersagemodell-Erzeugungseinheit (14A, 14B, 14C, 14D, 14E) eine vorbestimmte Gewichtungsberechnung an der Textindexwertgruppe durchführt, so dass ein durch die Gewichtungsberechnung erhaltener Wert sich einem bekannten Wert annähert, der den Schweregrad der Demenz darstellt, und das Vorhersagemodell erzeugt, um eine Ausgabe des Wertes zu erhalten, der den Schweregrad der Demenz aus der Textindexwertgruppe anzeigt, wobei ein gewichteter Wert für die Textindexwertgruppe als die Merkmalsgröße verwendet wird.

10. Demenzvorhersagevorrichtung nach einem der Ansprüche 3 bis 7, wobei die Lerndaten-Eingabeeinheit (10) als Lerndaten mehrere Texte eingibt, die Inhalte freier Konversationen darstellen, die von mehreren Patienten geführt werden, deren Schweregrad der Demenz jeweils bekannt ist, für jeweils mehrere Bewertungselemente der Demenz,

die Vorhersagemodell-Erzeugungseinheit (14A, 14B, 14C, 14D, 14E) ein Vorhersagemodell erzeugt, um eine Ausgabe des Wertes zu erhalten, der den Schweregrad für jedes der Bewertungselemente der Demenz basierend auf der Textindexwertgruppe anzeigt, und

die Demenzvorhersageeinheit (21A, 21B, 21C, 21D, 21E) die Ausgabe des Wertes erhält, der den Schweregrad für jedes der Bewertungselemente der Demenz für den der Vorhersage unterzogenen Patienten anzeigt.

11. Demenzvorhersagevorrichtung nach Anspruch 10, wobei die Vorhersagemodell-Erzeugungseinheit (14A, 14B, 14C, 14D, 14E) eine Merkmalsgröße berechnet, die mit dem Schweregrad für jedes der Bewertungselemente der Demenz für jedes der Bewertungselemente für die Textindexwertgruppe verbunden ist, und das Vorhersagemodell erzeugt, um eine Ausgabe des Wertes zu erhalten, der den Schweregrad für jedes der Bewertungselemente der Demenz aus der Textindexwertgruppe basierend auf der berechneten Merkmalsgröße anzeigt.

12. Demenzvorhersagevorrichtung nach Anspruch 11, **dadurch gekennzeichnet, dass** die Vorhersagemodell-Erzeugungseinheit (14A, 14B, 14C, 14D, 14E) eine vorbestimmte Gewichtungsberechnung an der Textindexwertgruppe durchführt, so dass ein durch die Gewichtungsberechnung für jedes der Bewertungselemente erhaltener Wert sich einem bekannten Wert annähert, der den Schweregrad für jedes der Bewertungselemente der Demenz anzeigt, und das Vorhersagemodell erzeugt, um eine Ausgabe des Wertes zu erhalten, der den Schweregrad für jedes der Bewertungselemente der Demenz aus der Textindexwertgruppe anzeigt, wobei ein gewichteter Wert für die Textindexwertgruppe als die Merkmalsgröße für jedes der Bewertungselemente verwendet wird.

13. Demenzvorhersagevorrichtung, die aufweist:

eine Vorhersagedaten-Eingabeeinheit (20), die einen oder mehrere Texte, die den Inhalt einer freien Konversation darstellen, die von einem der Vorhersage unterzogenen Patienten geführt wird, als Vorhersagedaten eingibt,
eine zweite Elementextraktionseinheit (11'), die Morpheme des einen oder der mehreren Texte, die von der Vorhersagedaten-Eingabeeinheit (20) als Vorhersagedaten eingegeben werden, analysiert und mehrere Wörter und/oder mehrere Sprachteile als mehrere Zerlegungselemente aus dem einen oder den mehreren Texten extrahiert, jeweils in Verbindung mit einer Erscheinungshäufigkeit;

eine zweite Textvektor-Berechnungseinheit (121'), die den einen oder die mehreren Texte in einen q-dimensionalen Vektor, wobei q eine beliebige ganze Zahl von 2 oder mehr ist, gemäß einer vorbestimmten Regel umwandelt, wodurch ein oder mehrere Textvektoren mit q Achsenkomponenten berechnet werden,

eine zweite Elementvektor-Berechnungseinheit (120'), die jedes der mehreren Zerlegungselemente gemäß einer vorbestimmten Regel in einen q-dimensionalen Vektor umwandelt, wodurch mehrere Elementvektoren berechnet werden, die q Achsenkomponenten enthalten,

eine zweite Indexwert-Berechnungseinheit (13'), die jeweils innere Produkte des einen oder der mehreren Textvektoren und der mehreren Elementvektoren erhält, wodurch Beziehungsindexwerte berechnet werden, die eine Beziehung zwischen dem einen oder den mehreren Texten und den mehreren Zerlegungselementen wiedergeben, und

eine Demenzvorhersageeinheit (21'), die einen Beziehungsindexwert, der von der zweiten Indexwert-Berechnungseinheit (13') berechnet wurde, auf ein Vorhersagemodell anwendet, das von der Vorhersagemodellerzeugungsvorrichtung nach Anspruch 1 erzeugt wurde, wodurch eine Ausgabe des Wertes erhalten wird, der den Schweregrad der Demenz für den der Vorhersage unterzogenen Patienten anzeigt,

wobei der Wert, der den Schweregrad der Demenz anzeigt, ein Wert einer Auswertung einer Mini-Geisteszustandsprüfung oder ein Wert ist, der eine Kategorie anzeigt, die durch eine Zahl größer als 2 und kleiner als ein Maximalwert der Auswertung der Mini-Geisteszustandsprüfung klassifiziert ist.

**14.** Demenzvorhersageprogramm, das einen Computer dazu veranlasst, zu funktionieren als:

eine Lerndaten-Eingabeeinrichtung, die mehrere Texte, die Inhalte von freien Konversationen repräsentieren, die von mehreren Patienten geführt werden, deren Schweregrad der Demenz jeweils bekannt ist, jeweils als Lerndaten eingibt,

eine Elementextraktionseinrichtung, die Morpheme der mehreren Texte, die durch die Lerndaten-Eingabeeinrichtung eingegeben werden, als die Lerndaten analysiert und mehrere Wörter und/oder mehrere Sprachteile als mehrere Zerlegungselemente aus den mehreren Texten extrahiert, jeweils in Verbindung mit einer Erscheinungshäufigkeit;

eine Textvektor-Berechnungseinrichtung, die jeden der mehreren Texte in einen q-dimensionalen Vektor, wobei q eine beliebige ganze Zahl von 2 oder mehr ist, gemäß einer vorbestimmten Regel umwandelt, wodurch mehrere Textvektoren mit q Achsenkomponenten berechnet werden,

eine Elementvektor-Berechnungseinrichtung, die jedes der mehreren Zerlegungselemente gemäß einer vorbestimmten Regel in einen q-dimensionalen Vektor umwandelt, wodurch mehrere Elementvektoren mit q Achsenkomponenten berechnet werden,

eine Indexwert-Berechnungseinrichtung, die jeweils innere Produkte der mehreren Textvektoren und der mehreren Elementvektoren erhält, wodurch Beziehungsindexwerte berechnet werden, die eine Beziehung zwischen den mehreren Texten und den mehreren Zerlegungselementen wiedergeben, und

eine Vorhersagemodell-Erzeugungseinrichtung, die ein maschinell erlerntes Vorhersagemodell erzeugt, so dass ein Wert, der den Schweregrad der Demenz anzeigt, ausgegeben wird, wenn eine Textindexwertgruppe, die mehrere Beziehungsindexwerte für einen Text enthält, unter Verwendung der Beziehungsindexwerte, die durch die Indexwert-Berechnungseinrichtung berechnet wurden, eingegeben wird,

wobei der Wert, der den Schweregrad der Demenz anzeigt, ein Wert einer Auswertung einer Mini-Geisteszustandsprüfung oder ein Wert ist, der eine Kategorie anzeigt, die durch eine Zahl größer als 2 und kleiner als ein Maximalwert der Auswertung der Mini-Geisteszustandsprüfung klassifiziert ist.

**15.** Programm zur Demenzvorhersage nach Anspruch 14, das den Computer ferner veranlasst, zu funktionieren als:

eine Vorhersagedaten-Eingabeeinrichtung, die einen Text, der den Inhalt einer freien Konversation repräsentiert, die von einem der Vorhersage unterzogenen Patienten geführt wurde, als Vorhersagedaten eingibt, und

eine Demenzvorhersageeinrichtung, die eine Ausgabe eines Wertes erhält, der den Schweregrad der Demenz für den der Vorhersage unterzogenen Patienten anzeigt, indem sie eine Textindexwertgruppe, die durch Ausführen von Prozessen der Elementextraktionseinrichtung, der Textvektor-Berechnungseinrichtung, der Elementvektor-Berechnungseinrichtung und der Indexwert-Berechnungseinrichtung an den durch die Vorhersagedateneingabeeinrichtung eingegebenen Vorhersagedaten erhalten wird, in das durch die Vorhersagemodell-Erzeugungseinrichtung erzeugte Vorhersagemodell eingibt.

**16.** Demenzvorhersageprogramm, das einen Computer dazu veranlasst, zu funktionieren als:

eine Vorhersagedaten-Eingabeeinrichtung, die einen oder mehrere Texte, die den Inhalt einer freien Konversation repräsentieren, die von einem der Vorhersage unterzogenen Patienten geführt wird, als Vorhersagedaten eingibt,

ein zweites Elementextraktionsmittel, das Morpheme des einen oder der mehreren Texte, die durch die Vorhersagedaten-Eingabeeinrichtung als die Vorhersagedaten eingegeben wurden, analysiert und mehrere Wörter und/oder mehrere Sprachteile als mehrere Zerlegungselemente aus dem einen oder den mehreren Texten extrahiert, jeweils in Verbindung mit einer Erscheinungshäufigkeit;

eine zweite Textvektor-Berechnungseinrichtung, die den einen oder die mehreren Texte in einen q-dimensionalen Vektor, wobei q eine beliebige ganze Zahl von 2 oder mehr ist, gemäß einer vorbestimmten Regel umwandelt, wodurch ein oder mehrere Textvektoren mit q Achsenkomponenten berechnet werden,

eine zweite Elementvektor-Berechnungseinrichtung, die jedes der mehreren Zerlegungselemente gemäß einer vorbestimmten Regel in einen q-dimensionalen Vektor umwandelt, wodurch mehrere Elementvektoren mit q Achsenkomponenten berechnet werden,

eine zweite Indexwert-Berechnungseinrichtung, die jeweils innere Produkte des einen oder der mehreren Textvektoren und der mehreren Elementvektoren erhält, wodurch Beziehungsindexwerte berechnet werden, die eine Beziehung zwischen dem einen oder den mehreren Texten und den mehreren Zerlegungselementen wiedergeben, und eine Demenzvorhersageeinrichtung, die einen durch die zweite Indexwert-Berechnungseinrichtung berechneten Beziehungsindexwert auf ein Vorhersagemodell anwendet, das durch die Vorhersagemodell-Erzeugungseinrichtung nach Anspruch 14 erzeugt wird, wodurch eine Ausgabe des Wertes erhalten wird, der den Schweregrad der Demenz für den der Vorhersage unterzogenen Patienten anzeigt.

## Revendications

1. Dispositif de génération de modèles de prédiction, comprenant :

une unité d'entrée de données d'apprentissage (10) qui applique en entrée une pluralité de textes représentant des contenus de conversations libres menées par une pluralité de patients dont la gravité de la démence est connue, respectivement, en tant que des données d'apprentissage ;

une unité d'extraction d'éléments (11A, 11B) qui analyse des morphèmes de la pluralité de textes appliquée en entrée par l'unité d'entrée de données d'apprentissage (10), en tant que les données d'apprentissage, et extrait au moins un élément parmi une pluralité de mots et une pluralité de parties de discours, en tant qu'une pluralité d'éléments de décomposition, de la pluralité de textes, chacun en association avec une fréquence d'apparition ;

une unité de calcul de vecteurs de texte (121) qui convertit chaque texte de la pluralité de textes en un vecteur à « q » dimensions, dans lequel « q » est un nombre entier arbitraire égal à 2 ou plus, selon une règle prédéterminée, ce qui permet de calculer par conséquent une pluralité de vecteurs de texte incluant « q » composantes d'axe ;

une unité de calcul de vecteurs d'éléments (122, 123) qui convertit chaque élément de la pluralité d'éléments de décomposition en un vecteur à « q » dimensions selon une règle prédéterminée, ce qui permet de calculer par conséquent une pluralité de vecteurs d'éléments incluant « q » composantes d'axe ;

une unité de calcul de valeurs d'index (13A, 13B, 13C) qui obtient chacun des produits intérieurs de la pluralité de vecteurs de texte et de la pluralité de vecteurs d'éléments, ce qui permet de calculer par conséquent des valeurs d'index de relation reflétant une relation entre la pluralité de textes et la pluralité d'éléments de décomposition ; et

une unité de génération de modèles de prédiction (14A, 14B, 14C, 14D, 14E) qui génère un modèle de prédiction ayant fait l'objet d'un apprentissage automatique de sorte qu'une valeur indiquant la gravité de la démence est fournie en sortie lorsqu'un groupe de valeurs d'index de texte incluant une pluralité de valeurs d'index de relation pour un texte est appliqué en entrée en utilisant les valeurs d'index de relation calculées par l'unité de calcul de valeurs d'index (13A, 13B, 13C) ;

dans lequel la valeur indiquant la gravité de la démence est une valeur d'un score d'un mini-examen de l'état mental ou une valeur indiquant une catégorie classée par un nombre supérieur à 2 et inférieur à une valeur maximale du score du mini-examen de l'état mental.

2. Dispositif de génération de modèles de prédiction selon la revendication 1,

dans lequel l'unité d'entrée de données d'apprentissage (10) applique en entrée, en tant que des données d'apprentissage, une pluralité de textes représentant des contenus de conversations libres menées par une pluralité de patients dont la gravité de la démence est connue, respectivement, pour chaque élément d'une pluralité d'éléments d'évaluation de la démence ; et

dans lequel l'unité de génération de modèles de prédiction (14A, 14B, 14C, 14D, 14E) génère un modèle de prédiction en vue d'obtenir une sortie de la valeur indiquant la gravité pour chacun des éléments d'évaluation de la démence sur la base du groupe de valeurs d'index de texte.

3. Dispositif de prédiction de la démence incluant le dispositif de génération de modèles de prédiction selon la revendication 1, et comprenant en outre :

une unité d'entrée de données de prédiction (20) qui applique en entrée un texte représentant un contenu d'une conversation libre menée par un patient soumis à une prédiction, en tant que des données de prédiction ; et une unité de prédiction de la démence (21A, 21B, 21C, 21D, 21E) qui obtient une sortie d'une valeur indiquant la gravité de la démence pour le patient soumis à une prédiction, en appliquant en entrée un groupe de valeurs d'index de texte obtenu en exécutant des processus de l'unité d'extraction d'éléments (11A, 11B), de l'unité de calcul de vecteurs de texte (121), de l'unité de calcul de vecteurs d'éléments (122, 123) et de l'unité de calcul de valeurs d'index (13A, 13B, 13C), sur les données de prédiction appliquées en entrée par l'unité d'entrée de données de prédiction (20) au modèle de prédiction généré par l'unité de génération de modèles de prédiction (14A, 14B, 14C, 14D, 14E).

4. Dispositif de prédiction de la démence selon la revendication 3, dans lequel :

l'unité d'entrée de données d'apprentissage (10) applique en entrée, en tant que les données d'apprentissage, « m » textes représentant des contenus de conversations libres menées par « m » patients, dans lequel « m » est un nombre entier arbitraire égal à 2 ou plus, dont la gravité de la démence est connue, respectivement ;

l'unité d'extraction d'éléments (11A, 11B) est une unité d'extraction de mots (11A) qui analyse les « m » textes appliqués en entrée en tant que les données d'apprentissage par l'unité d'entrée de données d'apprentissage (10) et extrait « n » mots, dans lequel « n » est un nombre entier arbitraire égal à 2 ou plus, des « m » textes, en association avec une fréquence d'apparition des mots ;

l'unité de calcul de vecteurs de texte (121) convertit chacun des « m » textes en un vecteur à « q » dimensions selon une règle prédéterminée, ce qui permet de calculer par conséquent « m » vecteurs de texte incluant « q » composantes d'axe ;

l'unité de calcul de vecteurs d'éléments (122, 123) est une unité de calcul de vecteurs de mots (122) qui convertit chacun des « n » mots en un vecteur à « q » dimensions selon une règle prédéterminée, ce qui permet de calculer par conséquent « n » vecteurs de mots incluant « q » composantes d'axe ;

l'unité de calcul de valeurs d'index (13A) obtient un produit d'une matrice de textes présentant les « q » composantes d'axe respectives des « m » vecteurs de texte, en tant que des éléments respectifs, et d'une matrice de mots présentant les « q » composantes d'axe respectives des « n » vecteurs de mots, en tant que des éléments respectifs, ce qui permet de calculer par conséquent une matrice de valeurs d'index présentant « m » x « n » valeurs d'index de relation reflétant une relation entre les « m » textes et les « n » mots, en tant que des éléments respectifs ;

l'unité de génération de modèles de prédiction (14A, 14D, 14E) génère un modèle de prédiction ayant fait l'objet d'un apprentissage automatique de sorte que la valeur indiquant la gravité de la démence est fournie en sortie lorsque le groupe de valeurs d'index de texte incluant « n » valeurs d'index de relation incluses dans la ligne identique de la matrice de valeurs d'index est appliqué en entrée en utilisant la matrice de valeurs d'index calculée par l'unité de calcul de valeurs d'index (13A) ;

l'unité d'entrée de données de prédiction (20) applique en entrée, en tant que des données de prédiction, « m' » textes représentant des contenus de conversations libres menées par « m' » patients, dans lequel « m' » est un nombre entier arbitraire égal à 1 ou plus, soumis à une prédiction, respectivement ; et

l'unité de prédiction de la démence (21A, 21D, 21E) obtient une sortie de la valeur indiquant la gravité de la démence pour les « m' » patients soumis à une prédiction, en appliquant en entrée « m' » groupes de valeurs d'index de texte obtenus en exécutant des processus de l'unité d'extraction de mots (11A), de l'unité de calcul de vecteurs de texte (121), de l'unité de calcul de vecteurs de mots (122) et de l'unité de calcul de valeurs d'index (13A), sur les données de prédiction appliquées en entrée par l'unité d'entrée de données de prédiction (20) au modèle de prédiction généré par l'unité de génération de modèles de prédiction (14A, 14D, 14E).

5. Dispositif de prédiction de la démence selon la revendication 3, dans lequel :

l'unité d'entrée de données d'apprentissage (10) applique en entrée, en tant que des données d'apprentissage, « m » textes représentant des contenus de conversations libres menées par « m » patients, dans lequel « m » est un nombre entier arbitraire égal à 2 ou plus, dont la gravité de la démence est connue, respectivement ;

l'unité d'extraction d'éléments (11A, 11B) est une unité d'extraction de parties de discours (11B) qui analyse les « m » textes appliqués en entrée en tant que les données d'apprentissage par l'unité d'entrée de données d'apprentissage (10) et extrait « p » parties de discours, dans lequel « p » est un nombre entier arbitraire égal à 2 ou plus, des « m » textes, en association avec une fréquence d'apparition des parties de discours ;

l'unité de calcul de vecteurs de texte (121) convertit chacun des « m » textes en un vecteur à « q » dimensions selon une règle prédéterminée, ce qui permet de calculer par conséquent « m » vecteurs de texte incluant « q » composantes d'axe ;

l'unité de calcul de vecteurs d'éléments (122, 123) est une unité de calcul de vecteurs de parties de discours (123) qui convertit les « p » parties de discours en un vecteur à « q » dimensions selon une règle prédéterminée, ce qui permet de calculer par conséquent « p » vecteurs de parties de discours incluant « q » composantes d'axe ;

l'unité de calcul de valeurs d'index (13B) obtient un produit d'une matrice de textes présentant les « q » composantes d'axe respectives des « m » vecteurs de texte, en tant que des éléments respectifs, et d'une matrice de parties de discours présentant les « q » composantes d'axe respectives des « p » vecteurs de parties de discours, en tant que des éléments respectifs, ce qui permet de calculer par conséquent une matrice de valeurs d'index présentant « m » x « p » valeurs d'index de relation reflétant une relation entre les « m » textes et les « p » parties de discours, en tant que des éléments respectifs ;

l'unité de génération de modèles de prédiction (14B, 14D, 14E) génère un modèle de prédiction ayant fait l'objet d'un apprentissage automatique de sorte que la valeur indiquant la gravité de la démence est fournie en sortie lorsque le groupe de valeurs d'index de texte incluant « p » valeurs d'index de relation incluses dans la ligne identique de la matrice de valeurs d'index est appliqué en entrée en utilisant la matrice de valeurs d'index calculée par l'unité de calcul de valeurs d'index (13B) ;

l'unité d'entrée de données de prédiction (20) applique en entrée, en tant que des données de prédiction, « m' » textes représentant des contenus de conversations libres menées par « m' » patients, dans lequel « m' » est un nombre entier arbitraire égal à 1 ou plus, soumis à une prédiction, respectivement ; et

l'unité de prédiction de la démence (21B, 21D, 21E) obtient une sortie de la valeur indiquant la gravité de la démence pour les « m' » patients soumis à une prédiction, en appliquant en entrée « m' » groupes de valeurs d'index de texte obtenus en exécutant des processus de l'unité d'extraction de parties de discours (11B), de l'unité de calcul de vecteurs de texte (121), de l'unité de calcul de vecteurs de parties de discours (123) et de l'unité de calcul de valeurs d'index (13B), sur les données de prédiction appliquées en entrée par l'unité d'entrée de données de prédiction (20), au modèle de prédiction généré par l'unité de génération de modèles de prédiction (14B, 14D, 14E).

6. Dispositif de prédiction de la démence selon la revendication 3, dans lequel :

l'unité d'entrée de données d'apprentissage (10) applique en entrée, en tant que les données d'apprentissage, « m » textes représentant des contenus de conversations libres menées par « m » patients, dans lequel « m » est un nombre entier arbitraire égal à 2 ou plus, dont la gravité de la démence est connue, respectivement ;

l'unité d'extraction d'éléments (11A, 11B) inclut une unité d'extraction de mots (11A) qui analyse les « m » textes appliqués en entrée, en tant que les données d'apprentissage, par l'unité d'entrée de données d'apprentissage (10), et extrait « n » mots, dans lequel « n » est un nombre entier arbitraire égal à 2 ou plus, des « m » textes, en association avec une fréquence d'apparition des mots, et une unité d'extraction de parties de discours (11B) qui analyse les « m » textes appliqués en entrée, en tant que les données d'apprentissage, par l'unité d'entrée de données d'apprentissage (10), et extrait « p » parties de discours, dans lequel « p » est un nombre entier arbitraire égal à 2 ou plus, des « m » textes, en association avec une fréquence d'apparition des parties de discours ;

l'unité de calcul de vecteurs de texte (121) convertit chacun des « m » textes en un vecteur à « q » dimensions selon une règle prédéterminée, ce qui permet de calculer par conséquent « m » vecteurs de texte incluant « q » composantes d'axe ;

l'unité de calcul de vecteurs d'éléments (122, 123) inclut une unité de calcul de vecteurs de mots (122) qui convertit chacun des « n » mots en un vecteur à « q » dimensions selon une règle prédéterminée, ce qui permet de calculer par conséquent « n » vecteurs de mots incluant « q » composantes d'axe, et une unité de calcul de vecteurs de parties de discours (123) qui convertit chacune des « p » parties de discours en un vecteur à « q » dimensions selon une règle prédéterminée, ce qui permet de calculer par conséquent « p » vecteurs de parties de discours incluant « q » composantes d'axe ;

l'unité de calcul de valeurs d'index (13C) obtient un produit d'une matrice de textes présentant les « q » composantes d'axe respectives des « m » vecteurs de texte, en tant que des éléments respectifs, et d'une matrice de mots présentant les « q » composantes d'axe respectives des « n » vecteurs de mots, en tant que des éléments respectifs, ce qui permet de calculer par conséquent une première matrice de valeurs d'index présentant « m » x « n » valeurs d'index de relation reflétant une relation entre les « m » textes et les « n » mots, en tant que des éléments respectifs, et obtient un produit de la matrice de textes présentant les « q » composantes d'axe respectives des vecteurs de texte, en tant que des éléments respectifs, et d'une matrice de parties de discours présentant les « q » composantes d'axe respectives des « p » vecteurs de parties de discours, en tant que des éléments respectifs, ce qui permet de calculer par conséquent une seconde matrice de valeurs d'index présentant « m » x « p » valeurs d'index de relation reflétant une relation entre les « m » textes et les « p » parties de discours, en tant que des éléments respectifs ;

l'unité de génération de modèles de prédiction (14C, 14D, 14E) génère un modèle de prédiction ayant fait l'objet d'un apprentissage automatique de sorte que la valeur indiquant la gravité de la démence est fournie en sortie lorsqu'un premier groupe de valeurs d'index de texte incluant « n » valeurs d'index de relation incluses dans la ligne identique de la première matrice de valeurs d'index et un second groupe de valeurs d'index de texte incluant « p » valeurs d'index de relation incluses dans la ligne identique de la seconde matrice de valeurs d'index sont appliqués en entrée en utilisant la première matrice de valeurs d'index et la seconde matrice de valeurs d'index calculées par l'unité de calcul de valeurs d'index (13C) ;

l'unité d'entrée de données de prédiction (20) applique en entrée, en tant que des données de prédiction, « m' » textes représentant des contenus de conversations libres menées par « m' » patients, dans lequel « m' » est un nombre entier arbitraire égal à 1 ou plus, soumis à une prédiction, respectivement ; et

l'unité de prédiction de la démence (21C, 21D, 21E) obtient une sortie de la valeur indiquant la gravité de la démence pour les « m' » patients soumis à une prédiction en appliquant en entrée « m' » premiers groupes de valeurs d'index de texte et « m' » seconds groupes de valeurs d'index de texte, obtenus en exécutant des processus de l'unité d'extraction de mots (11A), de l'unité d'extraction de parties de discours (11B), de l'unité de calcul de vecteurs de texte (121), de l'unité de calcul de vecteurs de mots (122), de l'unité de calcul de vecteurs de parties de discours (123) et de l'unité de calcul de valeurs d'index (13C), sur les données de prédiction appliquées en entrée par l'unité d'entrée de données de prédiction (20), au modèle de prédiction généré par l'unité de génération de modèles de prédiction (14C, 14D, 14E).

7. Dispositif de prédiction de la démence selon l'une quelconque des revendications 4 à 6, comprenant en outre :

une unité de compression dimensionnelle (15) qui met en oeuvre un traitement de compression dimensionnelle prédéterminé sur la matrice de valeurs d'index calculée par l'unité de calcul de valeurs d'index (13A, 13B, 13C), ce qui permet de calculer par conséquent une matrice de valeurs d'index à compression dimensionnelle ;

dans lequel l'unité de génération de modèles de prédiction (14A, 14B, 14C, 14D, 14E) génère un modèle de prédiction ayant fait l'objet d'un apprentissage automatique de sorte que la valeur indiquant la gravité de la démence est fournie en sortie lorsque le groupe de valeurs d'index de texte incluant la pluralité de valeurs d'index de relation incluses dans la ligne identique de la matrice de valeurs d'index est appliqué en entrée en utilisant une matrice de valeurs d'index à compression dimensionnelle par l'unité de compression dimensionnelle (15) ; et

dans lequel l'unité de prédiction de la démence (21A, 21B, 21C, 21D, 21E) applique en entrée le groupe de valeurs d'index de texte extrait de la matrice de valeurs d'index à compression dimensionnelle obtenue en exécutant en outre le traitement de l'unité de compression dimensionnelle (15) sur la matrice de valeurs d'index calculée par l'unité de calcul de valeurs d'index (13A, 13B, 13C), au modèle de prédiction généré par l'unité de génération de modèles de prédiction (14A, 14B, 14C, 14D, 14E), ce qui permet d'obtenir par conséquent une sortie de la valeur indiquant la gravité de la démence pour le patient soumis à une prédiction.

8. Dispositif de prédiction de la démence selon l'une quelconque des revendications 3 à 7, dans lequel l'unité de génération de modèles de prédiction (14A, 14B, 14C, 14D, 14E) calcule une quantité de caractéristiques associée à la gravité de la démence pour le groupe de valeurs d'index de texte, et génère le modèle de prédiction en vue d'obtenir une sortie de la valeur indiquant la gravité de la démence à partir du groupe de valeurs d'index de texte sur la base de la quantité de caractéristiques calculée.

9. Dispositif de prédiction de la démence selon la revendication 8, dans lequel l'unité de génération de modèles de prédiction (14A, 14B, 14C, 14D, 14E) met en oeuvre un calcul de pondération prédéterminé sur le groupe de valeurs d'index de texte de sorte qu'une valeur obtenue par un calcul de pondération s'approche d'une valeur connue représentant la gravité de la démence, et génère le modèle de prédiction en vue d'obtenir une sortie de la valeur indiquant la gravité de la démence à partir du groupe de valeurs d'index de texte, en utilisant une valeur pondérée pour le groupe de valeurs d'index de texte en tant que la quantité de caractéristiques.

10. Dispositif de prédiction de la démence selon l'une quelconque des revendications 3 à 7, dans lequel :

l'unité d'entrée de données d'apprentissage (10) applique en entrée, en tant que des données d'apprentissage, une pluralité de textes représentant des contenus de conversations libres menées par une pluralité de patients dont la gravité de la démence est connue, respectivement, pour chaque élément d'une pluralité d'éléments d'évaluation de la démence ;
l'unité de génération de modèles de prédiction (14A, 14B, 14C, 14D, 14E) génère un modèle de prédiction en vue d'obtenir une sortie de la valeur indiquant la gravité pour chacun des éléments d'évaluation de la démence sur la base du groupe de valeurs d'index de texte ; et
l'unité de prédiction de la démence (21A, 21B, 21C, 21D, 21E) obtient une sortie de la valeur indiquant la gravité pour chacun des éléments d'évaluation de la démence pour le patient soumis à une prédiction.

11. Dispositif de prédiction de la démence selon la revendication 10, dans lequel l'unité de génération de modèles de prédiction (14A, 14B, 14C, 14D, 14E) calcule une quantité de caractéristiques associée à une gravité pour chacun des éléments d'évaluation de la démence pour chacun des éléments d'évaluation du groupe de valeurs d'index de texte, et génère le modèle de prédiction en vue d'obtenir une sortie de la valeur indiquant la gravité pour chacun des éléments d'évaluation de la démence à partir du groupe de valeurs d'index de texte, sur la base de la quantité de caractéristiques calculée.

12. Dispositif de prédiction de la démence selon la revendication 11, **caractérisé en ce que** l'unité de génération de modèles de prédiction (14A, 14B, 14C, 14D, 14E) met en oeuvre un calcul de pondération prédéterminé sur le groupe de valeurs d'index de texte de sorte qu'une valeur obtenue par un calcul de pondération pour chacun des éléments d'évaluation s'approche d'une valeur connue représentant la gravité pour chacun des éléments d'évaluation de la démence, et génère le modèle de prédiction en vue d'obtenir une sortie de la valeur indiquant la gravité pour chacun des éléments d'évaluation de la démence à partir du groupe de valeurs d'index de texte, en utilisant une valeur pondérée pour le groupe de valeurs d'index de texte, en tant que la quantité de caractéristiques pour chacun des éléments d'évaluation.

13. Dispositif de prédiction de la démence comprenant :

une unité d'entrée de données de prédiction (20) qui applique en entrée un ou plusieurs textes représentant un contenu d'une conversation libre menée par un patient soumis à une prédiction, en tant que des données de prédiction ;
une seconde unité d'extraction d'éléments (11') qui analyse des morphèmes dudit un ou desdits plusieurs textes appliqués en entrée par l'unité d'entrée de données de prédiction (20), en tant que les données de prédiction, et extrait au moins un élément parmi une pluralité de mots et une pluralité de parties de discours, en tant qu'une pluralité d'éléments de décomposition, dudit un ou desdits plusieurs textes, chacun en association avec une fréquence d'apparition ;
une seconde unité de calcul de vecteurs de texte (121') qui convertit ledit un ou lesdits plusieurs textes en un vecteur à « q » dimensions, dans lequel « q » est un nombre entier arbitraire égal à 2 ou plus, selon une règle prédéterminée, ce qui permet de calculer par conséquent un ou plusieurs vecteurs de texte incluant « q » composantes d'axe ;
une seconde unité de calcul de vecteurs d'éléments (120') qui convertit chaque élément de la pluralité d'éléments de décomposition en un vecteur à « q » dimensions selon une règle prédéterminée, ce qui permet de calculer par conséquent une pluralité de vecteurs d'éléments incluant « q » composantes d'axe ;
une seconde unité de calcul de valeurs d'index (13') qui obtient chacun parmi des produits internes dudit un ou desdits plusieurs vecteurs de texte et de la pluralité de vecteurs d'éléments, ce qui permet de calculer par conséquent des valeurs d'index de relation reflétant une relation entre ledit un ou lesdits plusieurs textes et la pluralité d'éléments de décomposition ; et
une unité de prédiction de la démence (21') qui applique une valeur d'index de relation calculée par la seconde unité de calcul de valeurs d'index (13') à un modèle de prédiction généré par le dispositif de génération de modèles de prédiction selon la revendication 1, ce qui permet d'obtenir par conséquent une sortie de la valeur indiquant la gravité de la démence pour le patient soumis à une prédiction ;
dans lequel la valeur indiquant la gravité de la démence est une valeur d'un score d'un mini-examen de l'état mental ou une valeur indiquant une catégorie classée par un nombre supérieur à 2 et inférieur à une valeur maximale du score du mini-examen de l'état mental.

**14.** Programme de prédiction de la démence qui amène un ordinateur à fonctionner en tant que :

un moyen d'entrée de données d'apprentissage qui applique en entrée une pluralité de textes représentant des contenus de conversations libres menées par une pluralité de patients dont la gravité de la démence est connue, respectivement, en tant que des données d'apprentissage ;

un moyen d'extraction d'éléments qui analyse des morphèmes de la pluralité de textes appliqués en entrée par le moyen d'entrée de données d'apprentissage, en tant que les données d'apprentissage, et qui extrait au moins un élément parmi une pluralité de mots et une pluralité de parties de discours, en tant qu'une pluralité d'éléments de décomposition, de la pluralité de textes, chacun en association avec une fréquence d'apparition ;

un moyen de calcul de vecteurs de texte qui convertit chaque texte de la pluralité de textes en un vecteur à « q » dimensions, dans lequel « q » est un nombre entier arbitraire égal à 2 ou plus, selon une règle prédéterminée, ce qui permet de calculer par conséquent une pluralité de vecteurs de texte incluant « q » composantes d'axe ;

un moyen de calcul de vecteurs d'éléments qui convertit chaque élément de la pluralité d'éléments de décomposition en un vecteur à « q » dimensions selon une règle prédéterminée, ce qui permet de calculer par conséquent une pluralité de vecteurs d'éléments incluant « q » composantes d'axe ;

un moyen de calcul de valeurs d'index qui obtient chacun des produits intérieurs de la pluralité de vecteurs de texte et de la pluralité de vecteurs d'éléments, ce qui permet de calculer par conséquent des valeurs d'index de relation reflétant une relation entre la pluralité de textes et la pluralité d'éléments de décomposition ; et

un moyen de génération de modèles de prédiction qui génère un modèle de prédiction ayant fait l'objet d'un apprentissage automatique de sorte qu'une valeur indiquant la gravité de la démence est fournie en sortie lorsqu'un groupe de valeurs d'index de texte incluant une pluralité de valeurs d'index de relation pour un texte est appliqué en entrée en utilisant les valeurs d'index de relation calculées par le moyen de calcul de valeurs d'index ;

dans lequel la valeur indiquant la gravité de la démence est une valeur d'un score d'un mini-examen de l'état mental ou une valeur indiquant une catégorie classée par un nombre supérieur à 2 et inférieur à la valeur maximale du score du mini-examen de l'état mental.

**15.** Programme de prédiction de la démence selon la revendication 14, amenant en outre l'ordinateur à fonctionner en tant que :

un moyen d'entrée de données de prédiction qui applique en entrée un texte représentant un contenu d'une conversation libre menée par un patient soumis à une prédiction, en tant que des données de prédiction ; et

un moyen de prédiction de la démence qui obtient une sortie d'une valeur indiquant la gravité de la démence pour le patient soumis à une prédiction, en appliquant en entrée un groupe de valeurs d'index de texte obtenu en exécutant des processus du moyen d'extraction d'éléments, du moyen de calcul de vecteurs de texte, du moyen de calcul de vecteurs d'éléments et du moyen de calcul de valeurs d'index sur les données de prédiction appliquées en entrée par le moyen d'entrée de données de prédiction, au modèle de prédiction généré par le moyen de génération de modèles de prédiction.

**16.** Programme de prédiction de la démence qui amène un ordinateur à fonctionner en tant que :

un moyen d'entrée de données de prédiction qui applique en entrée un ou plusieurs textes représentant un contenu d'une conversation libre menée par un patient soumis à une prédiction, en tant que des données de prédiction ;

un second moyen d'extraction d'éléments qui analyse des morphèmes dudit un ou desdits plusieurs textes appliqués en entrée par le moyen d'entrée de données de prédiction, en tant que les données de prédiction, et qui extrait au moins un élément parmi une pluralité de mots et une pluralité de parties de discours, en tant qu'une pluralité d'éléments de décomposition, dudit un ou desdits plusieurs textes, chacun en association avec une fréquence d'apparition ;

un second moyen de calcul de vecteurs de texte qui convertit ledit un ou lesdits plusieurs textes en un vecteur à « q » dimensions, dans lequel « q » est un nombre entier arbitraire égal à 2 ou plus, selon une règle prédéterminée, ce qui permet de calculer par conséquent un ou plusieurs vecteurs de texte incluant « q » composantes d'axe ;

un second moyen de calcul de vecteurs d'éléments qui convertit chaque élément de la pluralité d'éléments de décomposition en un vecteur à « q » dimensions selon une règle prédéterminée, ce qui permet de calculer par conséquent une pluralité de vecteurs d'éléments incluant « q » composantes d'axe ;

un second moyen de calcul de valeurs d'index qui obtient chacun des produits intérieurs dudit un ou desdits plusieurs vecteurs de texte et de la pluralité de vecteurs d'éléments, ce qui permet de calculer par conséquent des valeurs d'index de relation reflétant une relation entre ledit un ou lesdits plusieurs textes et la pluralité d'éléments de décomposition ; et

un moyen de prédiction de la démence qui applique une valeur d'index de relation calculée par le second moyen de calcul de valeurs d'index, à un modèle de prédiction généré par le moyen de génération de modèles de prédiction selon la revendication 14, ce qui permet d'obtenir par conséquent une valeur indiquant la gravité de la démence pour le patient soumis à une prédiction.

**FIG. 1**

## FIG. 2

$$
\begin{pmatrix}
d\,w_{11} & d\,w_{12} & \cdots & d\,w_{1n} \\
d\,w_{21} & d\,w_{22} & \cdots & d\,w_{2n} \\
\vdots & \vdots & \ddots & \vdots \\
d\,w_{m1} & d\,w_{m2} & \cdots & d\,w_{mn}
\end{pmatrix}
$$

TEXT INDEX VALUE GROUP OF TEXT $d_1$

TEXT INDEX VALUE GROUP OF TEXT $d_m$

**FIG. 3**

(a) DURING LEARNING

START

INPUT m TEXTS AS LEARNING DATA — S1

EXTRACT n WORDS FROM m TEXTS — S2

COMPUTE TEXT VECTOR AND WORD VECTOR — S3

COMPUTE m × n RELATIONSHIP INDEX VALUES — S4

GENERATE PREDICTION MODEL FOR PREDICTING SEVERITY OF DEMENTIA FROM TEXT INDEX VALUE GROUP — S5

END

(b) DURING PREDICTION

START

INPUT m' TEXTS AS PREDICTION DATA — S11

EXTRACT n WORDS FROM m' TEXTS — S12

COMPUTE TEXT VECTOR AND WORD VECTOR — S13

COMPUTE m' × n RELATIONSHIP INDEX VALUES — S14

PREDICT SEVERITY OF DEMENTIA BY APPLICATION TO PREDICTION MODEL — S15

END

FIG. 4

RELATIONSHIP INDEX VALUE COMPUTATION APPARATUS ~100B

LEARNING DATA INPUT UNIT ~10

PART-OF-SPEECH EXTRACTION UNIT ~11B

VECTOR COMPUTATION UNIT ~12B

TEXT VECTOR COMPUTATION UNIT ~121

PART-OF-SPEECH VECTOR COMPUTATION UNIT ~123

INDEX VALUE COMPUTATION UNIT ~13B

PREDICTION MODEL GENERATION UNIT ~14B

PREDICTION DATA INPUT UNIT ~20

DEMENTIA PREDICTION UNIT ~21B

PREDICTION MODEL STORAGE UNIT ~30B

FIG. 5

(a)    I DO NOT KNOW WHAT I ATE LAST NIGHT.

(b)    I DO NOT KNOW WHAT I ATE LAST NIGHT.

## FIG. 6

| MAJOR CATEGORY | MEDIUM CATEGORY | MINOR CATEGORY | SUB-CATEGORY |
|---|---|---|---|
| NOUN | COMMON NOUN | GENERAL SA LINE TRANSFORMATION UTILIZATION IS ALLOWED | |
| | | ADJECTIVAL NOUN IS ALLOWED | |
| | | SA LINE TRANSFORMATION UTILIZATION AND ADJECTIVAL NOUN ARE ALLOWED | |
| | | ADVERB IS ALLOWED | |
| | PROPER NOUN | GENERAL | |
| | | NAME OF PERSON | GENERAL FAMILY NAME GIVEN NAME |
| | | PLACE NAME | GENERAL COUNTRY |
| | | NAME OF ORGANIZATION | |
| | NUMERAL | | |
| | AUXILIARY VERB STEM | | |
| PRONOUN | | | |
| ADJECTIVAL NOUN | GENERAL TARI AUXILIARY VERB STEM | | |
| ADNOMINAL ADJECTIVE | | | |
| ADVERB | | | |
| CONNECTIVE | | | |
| INTERJECTION | GENERAL FILLER | | |
| VERB | GENERAL CAN BE FUNCTIONAL | | |
| ADJECTIVE | GENERAL CAN BE FUNCTIONAL | | |
| AUXILIARY VERB | | | |
| POSTPOSITIONAL PARTICLE | CASE PARTICLE | | |
| | SUPPLEMENTARY PARTICLE | | |
| | BINDING PARTICLE | | |
| | CONJUNCTIVE PARTICLE | | |
| | SENTENCE-ENDING PARTICLE | | |
| | PARTICLE THAT ATTACHES TOPHRASE AND ACTS ON WHOLE PHRASE | | |
| PREFIX | | | |
| SUFFIX | NOUN SUFFIX | GENERAL SA LINE TRANSFORMATION UTILIZATION IS ALLOWED | |
| | | ADJECTIVAL NOUN IS ALLOWED | |
| | | ADVERB IS ALLOWED | |
| | | AUXILIARY NUMERAL | |
| | ADJECTIVAL NOUN SUFFIX | | |
| | VERB SUFFIX | | |
| | ADJECTIVE SUFFIX | | |

**FIG. 7**

## FIG. 8

**FIG. 9**

**FIG. 10A**

FIG. 10B

~100C

RELATIONSHIP INDEX VALUE COMPUTATION APPARATUS

~10
LEARNING DATA INPUT UNIT

~12C
VECTOR COMPUTATION UNIT

~121
TEXT VECTOR COMPUTATION UNIT

~122
WORD VECTOR COMPUTATION UNIT

~123
PART-OF-SPEECH VECTOR COMPUTATION UNIT

~11A
WORD EXTRACTION UNIT

~11B
PART-OF-SPEECH EXTRACTION UNIT

~13C
INDEX VALUE COMPUTATION UNIT

~15
DIMENSIONAL COMPRESSION UNIT

~20
PREDICTION DATA INPUT UNIT

~21C
DEMENTIA PREDICTION UNIT

~30C
PREDICTION MODEL STORAGE UNIT

~14C
PREDICTION MODEL GENERATION UNIT

44

FIG. 11

RELATIONSHIP INDEX VALUE COMPUTATION APPARATUS ～100A

LEARNING DATA INPUT UNIT ～10E

WORD EXTRACTION UNIT ～11A

VECTOR COMPUTATION UNIT ～12A

TEXT VECTOR COMPUTATION UNIT ～121

WORD VECTOR COMPUTATION UNIT ～122

INDEX VALUE COMPUTATION UNIT ～13A

PREDICTION MODEL GENERATION UNIT ～14E

PREDICTION DATA INPUT UNIT ～20

DEMENTIA PREDICTION UNIT ～21E

PREDICTION MODEL STORAGE UNIT ～30E

FIG. 12

RELATIONSHIP INDEX VALUE COMPUTATION APPARATUS 100'

PREDICTION DATA INPUT UNIT 20

SECOND ELEMENT EXTRACTION UNIT 11'

VECTOR COMPUTATION UNIT 12'

SECOND TEXT VECTOR COMPUTATION UNIT 121'

SECOND ELEMENT VECTOR COMPUTATION UNIT 120'

SECOND INDEX VALUE COMPUTATION UNIT 13'

DEMENTIA PREDICTION UNIT 21'

PREDICTION MODEL STORAGE UNIT 30'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002251467 A

### Non-patent literature cited in the description

- **SWETA KARLEKAR et al.** *Detecting Linguistic Characteristics of Alzheimer's Dementia by Interpreting Neural Models,* 17 April 2018, https://arxiv.org/abs/1804.06440

- Distributed Representations of Sentences and Documents. **QUOC LE ; TOMAS MIKOLOV.** Proceedings of the 31st International Conference on Machine Learning Held in Bejing. Google Inc